(19) Europäisches Patentamt
European Patent Office
Office européen des brevets

(11) EP 0 986 574 B1

(12) # EUROPÄISCHE PATENTSCHRIFT

(45) Veröffentlichungstag und Bekanntmachung des Hinweises auf die Patenterteilung:
**03.04.2002 Patentblatt 2002/14**

(21) Anmeldenummer: **98937433.5**

(22) Anmeldetag: **02.06.1998**

(51) Int Cl.[7]: **C07J 41/00**, A61K 31/565, C07J 53/00

(86) Internationale Anmeldenummer:
**PCT/EP98/03243**

(87) Internationale Veröffentlichungsnummer:
**WO 98/55499 (10.12.1998 Gazette 1998/49)**

(54) **11BETA-ARYLSUBSTITUIERTE 14,17-ETHANO ESTRATRIENE, VERFAHREN ZUR HERSTELLUNG DIESER VERBINDUNGEN, SOWIE IHRE VERWENDUNG ZUR HERSTELLUNG VON ARZNEIMITTELN**

11BETA-ARYL SUBSTITUTED 14,17-ETHANO-OESTRATRIENS, METHOD FOR THE PRODUCTION OF THESE COMPOUNDS AND THEIR USE IN THE PRODUCTION OF MEDICAMENTS.

14,17-ETHANO-OESTRATRIENES 11BETA-ARYL-SUBSTITUES, PROCEDE DE FABRICATION DE CES COMPOSES ET LEUR UTILISATION POUR LA FABRICATION DE MEDICAMENTS

(84) Benannte Vertragsstaaten:
**AT BE CH CY DE DK ES FI FR GB GR IE IT LI LU MC NL PT SE**
Benannte Erstreckungsstaaten:
**AL LT LV RO SI**

(30) Priorität: **02.06.1997 DE 19724187**

(43) Veröffentlichungstag der Anmeldung:
**22.03.2000 Patentblatt 2000/12**

(73) Patentinhaber: **Schering Aktiengesellschaft**
**13353 Berlin (DE)**

(72) Erfinder:
- **BOHLMANN, Rolf**
  **D-14055 Berlin (DE)**
- **FRITZEMEIER, Karl-Heinrich**
  **D-13505 Berlin (DE)**
- **HEGELE-HARTUNG, Christa**
  **D-10179 Berlin (DE)**
- **KNAUTHE, Rudolf**
  **D-13505 Berlin (DE)**
- **PARZCYK, Karsten**
  **D-12207 Berlin (DE)**

(56) Entgegenhaltungen:
**EP-A- 0 384 842**
**EP-A- 0 471 612**
**WO-A-93/13123**
**DE-A- 4 132 182**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 11β-arylsubstituierte 14,17-Ethanoestratriene der allgemeinen Formel I

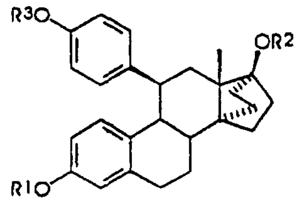

(I),

worin

$R^1$ für ein Wasserstoffatom, eine $C_1$-$C_{12}$-Alkanoyl-, eine Benzoyl-, eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe, die alle gegebenenfalls substituiert sein können,
$R^2$ für ein Wasserstoffatom oder eine gegebenenfalls substituierte $C_1$-$C_{12}$-Alkanoylgruppe und
$R^3$ für eine Gruppierung
$-(CH_2)_nNR^4R^5$ oder $-(CH_2)_nOCH_2CONR^4R^5$ oder $-(CH_2)_nS(O)_mR^6$
stehen,
worin
n 4, 5, 6 oder 7 ist,
m 0, 1 oder 2 ist,
$R^4$ oder $R^5$ unabhängig voneinander ein Wasserstoffatom oder eine geradkettige oder verzweigte, gegebenenfalls substituierte $C_1$-$C_8$-Alkylgruppe, die auch teilweise oder vollständig fluoriert sein kann, oder
$R^4$ und $R^5$ gemeinsam eine Methylen- bis Pentamethylengruppe $-(CH_2)_p-$ (p = 1 - 5) bedeuten und
$R^6$ die Bedeutung von $R^4$ oder $R^5$ hat oder ein gegebenenfalls substituierter Aryl- oder Arylalkylrest ist, wobei der Alkylrest darin bis zu 6 Kohlenstoffatome haben kann und der Arylrest, alleine oder im Arylalkylrest, ein 5- oder 6gliedriger monocyclischer Ring oder ein kondensiertes, 8 bis 10gliedriges Ringsystem ist und der Arylrest ein- oder mehrere Heteroatome ausgewählt unter Sauerstoff, Stickstoff und Schwefel aufweisen kann.

Bei den im Rahmen vorliegender Erfindung als $R^4$ und $R^5$ erwähnten Alkylgruppen handelt es sich beispielsweise um die Methyl-, Ethyl-, n- oder iso-Propyl-, n-, iso- oder tert.-Butyl-, Pentyl-, neo-Pentyl-, Hexyl-, Heptyl- oder Octylgruppe.
Bei der $C_1$-$C_{12}$-Alkylgruppe $R^1$ kommen noch die höheren Homologen wie beispielsweise die Nonyl- bis Dodecylgruppe hinzu.
Als $C_3$-$C_7$-Cycloalkylgruppe ist beispielsweise der Cyclopropyl-, Cyclopentyl-, Cyclohexyl- und Cycloheptylrest zu nennen.
Typische Vertreter für eine $C_4$-$C_8$-Alkylcycloalkylgruppe sind der Methylcyclopropyl-, Methylcyclopentyl- sowie der Methylcyclohexylrest.
Unter einem $C_1$-$C_{12}$-Alkanoylrest, der für $R^1$ und/oder $R^2$ stehen kann, werden die Acylreste der geradkettigen und verzweigten $C_1$-$C_{12}$-Alkancarbonsäuren verstanden, also beispielsweise der Formyl-, Acetyl-, Propionyl-, Butyryl- oder iso-Butyrylrest sowie die höheren Homologen vorstehend genannter Reste.
Als teilweise oder vollständig fluorierte geradkettige oder verzweigte $C_1$-$C_8$-Alkylgruppe ist beispielsweise die Trifluormethyl-, 2,2,2-Trifluorethyl-, 4,4,4-Trifluorbutyl-, 3,3,4,4,4-Pentafluorbutyl-, 4,4,5,5,5-Pentafluorpentyl- oder die Nonafluorbutylgruppe zu nennen.
Für den Arylrest $R^6$ oder den Arylrest innerhalb des Arylalkylrestes $R^6$ können die folgenden Reste stehen:

ein monocyclischer carbocyclischer Rest, beispielsweise der Phenylrest;
ein monocyclischer heterocyclischer Rest, beispielsweise der Thienyl-, Furyl-, Pyrrolyl-, Imidazolyl-, Pyrazolyl-, Pyridyl-, Pyrazinyl-, Pyrimidinyl-, Pyridazinyl-, Thiazolyl-, Oxazolyl-, Furazanyl-, Pyrrolinyl-, Imidazolinyl-, Pyrazolinyl-, Thiazolinyl-, Triazolyl-, Tetrazolylrest, und zwar alle möglichen Isomeren bezüglich der Positionen der Heteroatome sowie der Verknüpfungsstelle zum Schwefelatom in der Seitenkette;
ein kondensierter carbocyclischer Rest, beispielsweise der Naphthyl- oder Phenanthrenylrest, ein kondensierter Rest, der sich aus carbocyclischen und heterocyclischen Resten zusammensetzt, beispielsweise der Benzofuranyl-, Benzothienyl-, Benzimidazolyl-, Benzothiazolyl-, Naphto[2,3-b]thienyl-, Thianthrenyl-, Isobenzofuranyl-, Chromenyl-, Xanthenyl-, Phenoxathiinyl-, Indolizinyl-, Isoindolyl-, 3H-Indolyl, Indolyl-, Indazolyl-, Purinyl-, Chinolizinyl-, Isochinolyl-, Chinolyl-, Phthalazinyl-, Naphthyridinyl-, Chinoxalinyl Chinazolinyl-, Cinnolinyl-, Pteridinyl-, Carbazolyl-, β-Carbolinyl-, Acridinyl-, Phenazinyl-, Phenothiazinyl-, Phenoxazinyl-, Indolinyl-, Isoindolinyl-, Imidazopyridyl-, Imidazopyrimidinyl-oder
ein kondensiertes polyheterocyclisches System, beispielsweise Furo[2,3-b]pyrrol oder Thieno[2,3-b]furan.

Als Substituenten an den Resten $R^1$, $R^2$, $R^4$, $R^5$ und $R^6$ kommen die nachstehenden Substituenten in betracht, wobei die Reste einfach oder mehrfach, identisch oder unterschiedlich, mit diesen Substituenten substituiert sein können:

Halogenatome: Fluor, Chlor, Brom, Iod;
Amino-, Alkylamino- oder Dialkylaminogruppen, worin der Alkylrest 1 bis 4 Kohlenstoffatome hat; insbesondere Methylamino oder Ethylamino, Dimethylamino, Diethylamino oder Methylethylamino;
Hydroxylgruppen;
freie, veresterte oder in Form eines Salzes vorliegende Carboxylgruppen: verestert mit einer Carboxycarbonylgruppe, beispielsweise Methoxycarbonyl oder Ethoxycarbonyl; als Salz beispielsweise in Form des Natrium- oder Kaliumsalzes;
Alkylgruppen mit 1 bis 8 Kohlenstoffatomen, wie beispielsweise die Methyl-, Ethyl-, n- oder iso-Propyl-, n-, iso- oder tert.-Butylgruppe, gegebenenfalls substituiert mit einem oder mehreren Halogenatomen, zum Beispiel mit Fluor wie die Trifluormethylgruppe;
Oxo-, Cyano-, Nitro- oder Formylgruppen;
Acylgruppen wie Acetyl, Propionyl, Butyryl, Benzoyl;
Acyloxygruppen wie Acetoxy, Reste der Formel-O-CO-$(CH_2)_n$-COOH mit n = 1 bis 5; Alkoxygruppen wie zum Beispiel Methoxy, Ethoxy, Propoxy, Isopropoxy, Butoxy; Alkylthiogruppen, beispielsweise Methylthio, Ethylthio, Propylthio, Isopropylthio, Butylthio; Carbamoylgruppen;
Alkenylgruppen, beispielsweise Vinyl, Propenyl;
Alkinylgruppen, beispielsweise Ethinyl, Propinyl;
Arylgruppen wie Phenyl, Furyl, Thienyl.

$R^1$ und $R^2$ stehen vorzugsweise jeweils für ein Wasserstoffatom.
Soll $R^1$ und/oder $R^2$ eine Alkylgruppe sein, ist eine Methyl- oder Ethylgruppe bevorzugt. Steht $R^1$ und/oder $R^2$ für eine Alkanoylgruppe, ist in erster Linie an eine Acetyl- oder Propionylgruppe gedacht. Auch eine Benzoylgruppe gehört zu den bevorzugten Vertretern für diese Substituenten.
Als Cycloalkylgruppe für die Substituenten $R^1$ und $R^2$ ist vor allem die Cyclopropyl- und Cyclopentylgruppe und als Alkylcycloalkylgruppe die Methylcyclopropyl- und Methylcyclopentylgruppe zu nennen.
Innerhalb der für $R^3$ stehenden Gruppierungen sind für $R^4$ und $R^5$ die Substituenten Wasserstoff, Methyl, Ethyl, Propyl, Butyl, 4,4,5,5,5-Pentafluorpentyl und vor allem die Substituentenkombinatiorien 2 Wasserstoffatome, n-Butyl/Methyl,-$(CH_2)_4$-, -$(CH_2)_5$- bevorzugt.
$R^6$ steht vorzugsweise für eine n-Pentyl-, 4,4,5,5,5-Pentafluorpentyl- oder -$CH_2$-2-, 3- oder 4-Pyridingruppe.
Insbesondere steht $R^3$ für einen der folgenden Substituenten:

$$-(CH_2)_5S(CH_2)_4CH_3$$

$$-(CH_2)_5SO(CH_2)_4CH_3$$

$$-(CH_2)_5SO_2(CH_2)_4CH_3$$

$$-(CH_2)_5SO(CH_2)_3CF_2CF_3$$

$$-(CH_2)_5SO_2\text{-}CH_2\text{-(2-Pyridyl)}$$

$$-(CH_2)_5SO_2\text{-}CH_2\text{-(3-Pyridyl)}$$

$-(CH_2)_5SO_2-CH_2-$(4-Pyridyl)

$-(CH_2)_6-O-CH_2C(O)N(CH_3)C_4H_9$

$-(CH_2)_5SO_2(CH_2)_3CF_2CF_3$

$-(CH_2)_5SO-CH_2-$(2-Pyridyl)

$-(CH_2)_5SO-CH_2-$(3-Pyridyl)

$-(CH_2)_5SO-CH_2-$(4-Pyridyl)

$-(CH_2)_5-$Pyrrolidinyl

$-(CH_2)_6S(CH_2)_3CF_2CF_3$

$-(CH_2)_6SO(CH_2)_3CF_2CF_3$

$-(CH_2)_6SO_2(CH_2)_3CF_2CF_3$

Im Sinne der Erfindung besonders bevorzugte Verbindungen sind:

14α,17α-Ethano-11β-{4-[5-(pyrrolidin-1-yl)pentyloxy]phenyl}-1,3,5(10)-estratrien-3,17β-diol
14α,17α-Ethano-11β-{4-[5-(pentylthio)pentyloxy]phenyl}-1,3,5(10)-estratrien-3,17β-diol
14α,17α-Ethano-11β-{4-[5-(pentansulfinyl)pentyloxy]phenyl}-1,3,5(10)-estratrien-3,17β-diol
14α,17α-Ethano-11β-{4-[5-(pentansulfonyl)pentyloxy]phenyl}-1,3,5(10)-estratrien-3,17β-diol
14α,17α-Ethano-11β-{4-[5-(4,4,5,5,5-pentafluorpentylsulfinyl)pentyloxy]phenyl}-1,3,5(10)-estratrien-3,17β-diol
14α,17α-Ethano-11β-{4-[5-(2-pyridinmethylsulfonyl)pentyloxy]phenyl}-1,3,5(10)-estratrien-3,17β-diol
14α,17α-Ethano-11β-{4-[6-(4,4,5,5,5-pentafluorpentylthio)hexyloxy]-phenyl}-1,3,5(10)-estratrien-3,17β-diol
14α,17α-Ethano-11β-{4-[6-(4,4,5,5,5-pentafluorpentyl)sulfinyl]hexyloxy}phenyl-1,3,5(10)-estratrien-3,17β-diol
14α,17α-Ethano-11β-{4-[6-(4,4,5,5,5-pentafluorpentyl)sulfonyl]hexyloxy}phenyl-1,3,5(10)-estratrien-3,17β-diol
14α,17α-Ethano-11β-{4-[5-(2-pyridinmethylsulfinyl)pentyloxy]phenyl}-1,3,5(10)-estratrien-3,17β-diol
14α,17α-Ethano-11β-{4-[5-(4,4,5,5,5-pentafluorpentyl)sulfonyl]pentyloxy}phenyl-1,3,5(10)-estratrien-3,17β-diol
2-(6-(4-[10-{14,17-Ethano-3,17-dihydroxyestra-1,3,5(10)-trien-11β-yl}]-phenoxy)-hexyloxy)essigsäure-(N-butyl-N-methyl)-amid

Als den neuen Verbindungen der allgemeinen Formel I nächstkommende Verbindungen sind die in der WO-A 93/13123 beschriebenen 19-Nor Steroide mit einer 11β-Seitenkette der Formel $-X-Y-S(O)_m-Z$ und die in der EP-A 0 384 842 beschriebenen 19-Nor-Steroide mit einer 11β-Seitenkette der Formel -X-Y-Z-C(O)-N(RA)(RA') anzusehen. X, Y Z, RA und RA' können dabei so gewählt sein, daß diese Seitenketten mit den 11β-Substituenten gemäß vorliegender Erfindung, worin $R^3$ die Gruppierung $-(CH_2)_nOCH_2CONR^4R^5$ bzw.$-(CH_2)_nS(O)_mR^6$ bedeutet, identisch sind. Die Verbindungen des Standes der Technik verfügen aber nicht über die in den erfindungsgemäßen Verbindungen vorhandene 14α,17α-Ethanobrücke. Bei den bekannten Verbindungen kann es sich u.a. um Verbindungen mit estrogenen oder antiestrogenen Eigenschaften handeln. Die Verbindungen können dabei frei von uterotropher Wirkung sein. Andererseits sind 14α,17α-Ethanoestratriene, die in der 11β-Position einen endständig mit einer Amid-, Amin- oder Sulfinylgruppe substituierten Nonylrest aufweisen, in der WO 93/06124 beschrieben. Diese Verbindungen sind ausschließlich als Verbindungen mit besonders hoher Affinität zum Estrogenrezeptor beschrieben, die, auch nach peroraler Applikation, reine Antiestrogene mit starker antiestrogener Wirkung sind. Diese Verbindungen unterscheiden sich von

den vorliegenden durch das Substitutionsmerkmal in 11β-Position des Steroidgerüsts.

Bei den erfindungsgemäßen Verbindungen handelt es sich um selektive Estrogene, deren Wirkung gewebeselektiv auftritt. Insbesondere tritt die estrogene Wirkung am Knochen, im Herz-Kreislaufsystem und im ZNS (Zentrales Nervensystem) auf. Am Uterus und in der Leber tritt dagegen keine oder nur eine geringfügige estrogene Wirkung auf. Die Verbindungen können auch über antiestrogene Wirksamkeit verfügen, die sich beispielsweise im Antiuteruswachstumstest oder in Tumormodellen nachweisen läßt

Verbindungen mit einem derartigen Profil werden neuerdings als Selective Estrogen Receptor Modulators (SERMs) bezeichnet (Structure-Activity Relationships of Selective Estrogen Receptor Modulators: Modifications to the 2-Arylbenzothiophene Core of Raloxifene, T. A. Grese et al, J. Med. Chem. 1997, 40, 146 - 167). Prominentester Vertreter dieser Verbindungsklasse ist das Raloxifen, welches sich gegenwärtig in klinischer Evaluierung für die Prävention und die Behandlung der postmenopausalen Osteoporose befindet.

Pharmakologische Untersuchung der erfindungsgemäßen Verbindungen

Antiestrogene/estrogene Wirkung in vitro

Die antiestrogene Wirkung der erfindungsgemäßen Verbindungen wurde in Transaktivierungsassays bestimmt [Demirpence E., Duchesne M.-J., Badia E., Gagne D. und Pons M.: MVLN Cells: A Bioluminescent MCF-7-Derived Cell Line to study the Modulation of Estrogenic Activity; J. Steroid. Molec. Biol. Vol. 46, No. 3, 355 - 364 (1993) sowie Berry M., Metzger D. Chambon P.: Role of the two activating domains of the estrogen receptor in the cell-type and promoter-context dependent agonistic activity of the anti-estrogen 4-hydroxytamoxifen; The EMBO Journal Vol. 9, 2811 - 2818 (1990)].

Die Transaktivierungstests erfolgten in Hela- oder MVLN-Zellen.

Die Hela-Zellen sind transient mit humanem Estrogenrezeptor-Expressionsvektor (HEGO) und Vit-TK-CAT Reportergen und die MVLN-Zellen stabil mit dem Reportergen Vit-TK-LUC transfiziert. Es wurde die antiestrogene Wirkstärke in Gegenwart von 0,1 nM Estradiol bestimmt.

Die estrogene Partialwirkung wurde ebenfalls durch Transaktivierungsassays bestimmt.

HeLa-Zellen wurden mit Estrogenrezeptor und einem Reportergen transfiziert, das aus dem Estrogen-Response Element des Progesteronrezeptors und dem CAT-Gen besteht. Der Test wurde durchgeführt wie beschrieben: Savouret et al., EMBO J. 10, 1875 -1883 (1991).

Die Ergebnisse für die erfindungsgemäßen Verbindungen 14α,17α-Ethano-11β-{4-[5-(2-pyridinmethylsulfonyl)pentyloxy]phenyl}-1,3,5(10)-estratrien-3,17β-diol (Verbindung A) und 14α,17α-Ethano-11β-{4-[5-(2-pyridinmethylsulfinyl)pentyloxy]phenyl}-1,3,5(10)-estratrien-3,17β-diol (Verbindung B) sowie für 11β-{4-[5-(2-pyridinmethylsulfinyl) pentyloxy]phenyl}-1,3,5(10)-estratrien-3,17β-diol (Verbindung V) gemäß WO 93/13123 gehen aus der nachstehenden Tabelle 1 hervor. Verbindung V aus dem Stand der Technik unterscheidet sich von der Verbindung B lediglich durch das Nichtvorhandensein der 14α,17α-Ethanobrücke.

Der Einfluß der erfindungsgemäßen Verbindungen auf den Uterus wurde im Uteruswachstumstest (estrogene Wirkung) und im Antiuteruswachstumstest (antiestrogene Wirkung), beide an der infantilen Ratte durchgeführt, untersucht.

Estrogene/antiestrogene Wirkung in vivo

Uteruswachstumstest an der infantilen Ratte (n=5 Tiere/Gruppe)

Sowohl Uterus wie auch Vagina zeigen bei infantilen Tieren bei deren Behandlung mit einer estrogen wirksamen Substanz eine von der estrogenen Wirksamkeit abhängige Gewichtszunahme. Am Uterus kommt es unter estrogener Wirkung zudem zu einer Proliferation und Höhenzunahme des luminalen Epithels.

Immature, intakte Ratten (Körpergewicht 40-50g) erhalten über 3 Tage (d1-d3) die Substanz s.c.. Am Tag 4 (d4) werden die Tiere mit $CO_2$ getötet. Die Uteri werden herauspräpariert und gewogen. Ein Uterusstück, vorzugsweise ein Uterushorn, wird für die histologische Auswertung in Formaldehyd fixiert und in Paraffin eingebettet. Die Stimulierung der Organgewichte (bezogen auf mg/100g Körpergewicht) sowie die Epithelhöhe wird in prozentualer Stimulierung im Vergleich zur Referenzverbindung 17β-Estradiol angegeben. (Substitutiondosis $E_2$ 0,3 μg/Tier).

Die Ergebnisse für die erfindungsgemäßen Verbindungen 14α,17α-Ethano-11β-{4-[5-(2-pyridinmethylsulfonyl)pentyloxy]phenyl}-1,3,5(10)-estratrien-3,17β-diol (Verbindung A) und 14α,17α-Ethano-11β-{4-[5-(2-pyridinmethylsulfinyl)pentyloxy]phenyl}-1,3,5(10)-estratrien-3,17β-diol (Verbindung B) sowie für 11β-{4-[5-(2-pyridinmethylsulfinyl) pentyloxy]phenyl}-1,3,5(10)-estratrien-3,17β-diol (Verbindung V) gemäß WO 93/13123 gehen aus der nachstehenden Tabelle 2 hervor. Verbindung V aus dem Stand der Technik unterscheidet sich von der Verbindung B lediglich durch das Nichtvorhandensein der 14α,17α-Ethanobrücke.

Die Verbindung A stellt nach s.c. Applikation eine Verbindung ohne jegliche estrogene Wirkung am Uterus dar. Die Verbindungen B und V stellen sich im Uteruswachstumstest beide als Verbindungen mit geringer estrogener Wirkung dar, wobei die Verbindung B im betrachteten Dosisbereich etwa halb so stark estrogen wirksam ist wie die analoge Verbindung V ohne die $C_2$-Überbrückung. Diese Verhältnisse sind in der Tabelle 2 und der Figur 1 wiedergegeben.

Antiuteruswachstumstest an der infantilen Ratte (n=5 Tiere/Gruppe)

Der Uterus infantiler estrogensubstituierter Ratten kann als Testmodell genutzt werden, um eine direkte Wirkung von Substanzen mit antiestrogenen Eigenschaften nachzuweisen. Der Parameter der Estrogenwirkung ist das bei infantilen Ratten durch Estradiol induzierte Uteruswachstum, das durch gleichzeitige Gabe einer Substanz mit antiöstogener Wirkung gehemmt wird.
Die Testsubstanzen werden s.c. an 3 aufeinanderfolgenden Tagen (d1-d3) in Kombination mit einer Substitutionsdosis 0.3 µg/Tier/Tag 17β-Estradiol behandelt. Als Positivkontrolle dient 17β-Estradiol alleine, als Negativkontrolle die Vehikel-Gruppe. An Tag 4 (d4) werden die Tiere getötet, Uteri und Vaginae werden herauspräpariert und gewogen. Die Organgewichte werden auf mg/100 g Körpergewicht umgerechnet, dann der Mittelwert und die Standardabweichung für jede Dosierung berechnet. Die Hemmung des durch 17β-Estradiol induzierten Uterus- bzw. Vaginalwachstums wird als Hemmung in % angegeben.
Die Ergebnisse für die erfindungsgemäßen Verbindungen 14α,17α-Ethano-11β-{4-[5-(2-pyridinmethylsulfonyl)pentyloxy]phenyl}-1,3,5(10)-estratrien-3,17β-diol (Verbindung A) und 14α,17α-Ethano-11β-{4-[5-(2-pyridinmethylsulfinyl)pentyloxy]phenyl}-1,3,5(10)-estratrien-3,17β-diol (Verbindung B) sowie für 11β-{4-[5-(2-pyridinmethylsulfinyl) pentyloxy]phenyl}-1,3,5(10)-estratrien-3,17β-diol (Verbindung V) gemäß WO 93/13123 gehen aus der nachstehenden Tabelle 3 hervor. Verbindung V aus dem Stand der Technik unterscheidet sich von der Verbindung B lediglich durch das Nichtvorhandensein der 14α,17α-Ethanobrücke.
Alle drei getesteten Verbindungen sind demnach am Uterus stark antiestrogen wirksam.
Die erfindungsgemäßen Verbindungen sind somit hinsichtlich ihrer Wirkung am Uterus den Verbindungen des Standes der Technik im Sinne vorliegender Erfindung dahingehend überlegen, daß sie an diesem Organ geringere oder gar keine estrogene Wirkung aufweisen.

Knochenuntersuchungen

Methode

3 Monate alte weibliche Ratten werden ovarektomiert und unmittelbar nach der Operation 28 Tage lang 1mal täglich mit der Testverbindung behandelt. Die Applikation erfolgt subcutan in Rizinusöl/Benzylbenzoat oder Arachisöl/Ethanol. Die Tiere werden am Tag nach der letzten Applikation getötet und Femur, Tibia sowie die Uteri entnommen. Die Uteri werden gewogen, fixiert und für histologische Untersuchungen aufgearbeitet. Die Bestimmung der Knochendichte erfolgt ex vivo an präparierten Langknochen mittels pQCT (Quantitative Computertomographie). Die Messungen werden im Abstand von 5 - 7 mm vom Gelenkkopf am distalen Femur oder der proximalen Tibia durchgeführt.
Alternativ wird die Wirkung am Knochen durch Ausmessen der trabekulären Knochenfläche der sekundären Spongiosa an histologischen Präparaten des distalen Femur oder der proximalen Tibia festgestellt. Das Ergebnis wird als prozentualer Anteil der trabekulären Knochenfläche an der ausgemessenen Gesamtknochenfläche ausgedrückt (TB/BV).
Die mittels QCT gemessene Knochendichte und die am histologischen Schnitt ermittelte trabekuläre Knochenfläche korrelieren gut miteinander. Ein Vergleich beider Messgrößen ist deshalb zulässig.
Durch die Ovarektomie geht etwa 50% der Knochendichte der Tiere verloren. Durch die Behandlung mit einer Verbindung der allgemeinen Formel I gemäß vorliegender Erfindung (Verbindung A) wird der Abbau der Knochendichte verhindert bzw. gehemmt.
In Tabelle 4 und Figur 2 sind die Ergebnisse dieses Versuchs für Dosierungen von 0.1 - 100 µg/Tier/Tag wiedergegeben. Gemessen wurde die Knochendichte am distalen Femur; zum Vergleich wurde die trabekuläre Knochenfläche in der proximalen Tibia gemessen. Die in der Tabelle 4 enthaltenen Angaben bedeuten im einzelnen:

| | |
|---|---|
| sham: | scheinoperierte Tiere; diese Tiere sind nicht ovarektomiert, sie wurden nur geritzt, um den Operationseffekt für die Kontrollgruppe zu erzeugen |
| ovx: | ovarektomierte Gruppe |
| BMD-qCT | Bone Mineral Density, ermittelt durch quantitative Computertomographie; mittels dieses Verfahrens wird die Calciumdichte in einem Schnitt durch den Knochen als Indikator für die Knochendichte gemessen |
| TB/TV | trabekuläre Knochenfläche/Gesamtknochenfläche; histologisch ermittelt |

Die Verbindung A hat im Dosisbereich zwischen 10 und 100 µg eine protektive Wirkung von 50 - 60% im Vergleich zu 17β-Estradiol. Wie aus Figur 3 hervorgeht, stimuliert die Verbindung A im Gegensatz zum 17β-Estradiol das Uterusgewicht jedoch bestenfalls marginal.

In einem weiteren Versuch wurde die knochenprotektive Wirkung ebenfalls der Verbindung A nunmehr in Dosierungen von 100 - 2000 µg/Tier/Tag bestimmt. Die Knochendichte wurde dabei an der proximalen Tibia gemessen. Die Ergebnisse sind in der Tabelle 5 und in Figur 4 gezeigt. Die Bedeutung der Abkürzungen ist wie bei der Tabelle 4 bereits angegeben.

Verbindung A hat bei einer Dosis von 100µg/Tier/Tag eine Wirkung von ca. 80% im Vergleich zu 17β-Estradiol. Diese Wirkung läßt sich durch Erhöhung der Dosis nicht mehr steigern. Wie aus Figur 5 hervorgeht stimuliert die Verbindung A auch in diesem Versuch in allen getesten Dosierungen das Uterusgewicht nur marginal.

Für die erfindungsgemäße Verbindung B wurde ebenfalls deren knochenprotektive Wirkung bestimmt (Tabelle 6). Es wurde hierzu der Parameter TB/TV ermittelt, der jedoch hinsichtlich der Aussagekraft für die knochenprotektive Wirksamkeit mit dem für die Verbindungen A und V ermittelten Parameter BMD-qCT gut korreliert. Dies läßt sich der Tabelle 4 entnehmen; für Verbindung A wurden beide Parameter ermittelt.

Bei abgeschwächter oder nicht vorhandener uterotropher Estrogenwirkung weisen die erfindungsgemäßen Verbindungen gleichzeitig hinsichtlich ihrer knochenprotektiven Wirkung eine den nächstliegenden Verbindungen des Standes der Technik ähnliche oder sogar bessere Wirkung auf. Bei vergleichbarer oder besserer Knochenwirkung stimulieren die erfindungsgemäßen Verbindungen im Gegensatz zu den bekanten Verbindungen den Uterus weniger oder gar nicht.

Die erfindungsgemäßen Verbindungen sind also im Sinne einer selektiven Wirkung am Knochen oder zumindest hinsichtlich einer abgeschwächten Wirkung am Uterus deutlich stärker dissoziiert als die nächstligenden Verbindungen des Standes der Technik.
Dies zeigt der in Tabelle 7 und Figur 6 angegebene Vergleich mit der Verbindung V des Standes der Technik. Verbindung A hat bei 100µg/Tier/Tag eine Wirkung von ca. 80 % von 17β-Estradiol am trabekulären Knochen der proximalen Tibia. Diese Wirkung wird erst mit 250µg/Tier/Tag der Vergleichsverbindung V erzielt. Die erfindungsgemäße Verbindung A ist somit hinsichtlich der knochenprotektiven Wirkung um den Faktor 3 potenter als die Vergleichsverbindung V.

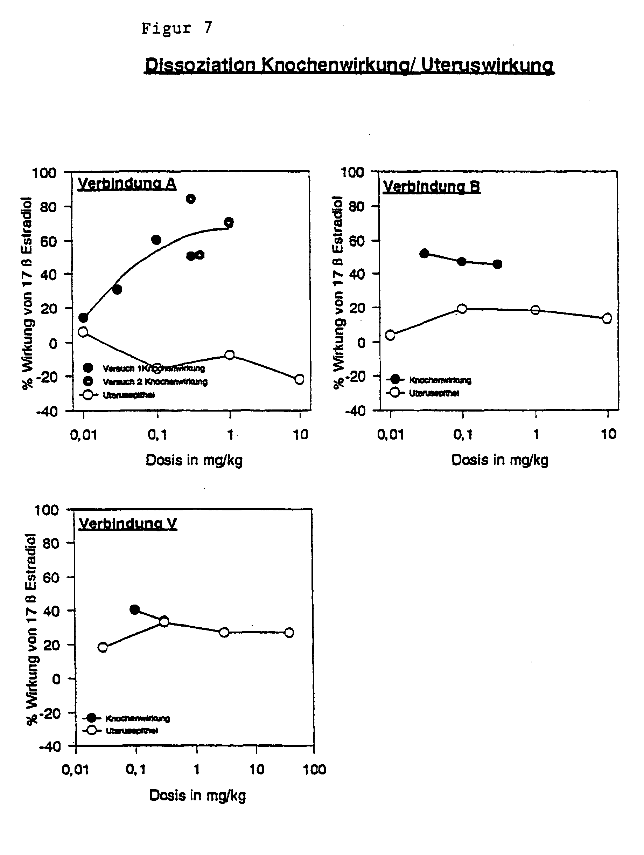

Die vorstehend beschriebenen Verhältnisse sind alle in der Figur 7 zusammengefaßt. Dort sind für die Verbindungen A, B und V deren knochenprotektive Wirkung der estrogenen Wirkung am Uterus (Parameter: Uterusepithelhöhe) in Abhängigkeit von der Dosierung, jeweils bezogen auf die Wirkung von 17β-Estradiol, einander gegenüber gestellt. Die Verbindung A ist an stärksien dissoziiert, die Verbindung B ist ebenfalls deutlich dissoziiert, während für die Verbindung V die beiden Kurven zusammenfallen.

Die Verbindungen können, sowohl nach oraler als auch parenteraler Gabe, für die folgenden Indikationen eingesetzt werden:
Linderung der Symptome der Andropause und Menopause, d.h. zur männlichen und weiblichen Hormonersatz-Therapie (HRT), und zwar sowohl zur Prävention als auch zur Behandlung; zur Behandlung der mit einer Dysmenorrhoe einhergehenden Beschwerden; Behandlung dysfunktioneller uteriner Blutungen; Behandlung der Akne; Prävention und Behandlung kardiovaskulärer Erkrankungen; Behandlung von Hypercholesterinämie und Hyperlipidämie; Prävention und Behandlung der Artherosclerose; zur Hemmung der Proliferation der arteriellen Glattmuskelzellen; zur Behandlung des Atemnotsyndroms bei Neugeborenen; Behandlung des primären pulmonaren Bluthochdrucks; zur Vorbeugung und Behandlung der Osteoporose; zur Vorbeugung des Knochenverlusts bei postmenopausalen Frauen, bei hysterektomierten Frauen oder bei Frauen, die mit LHRH Agonisten oder Antagonisten behandelt wurden; Hemmung der Spermienreifung; Behandlung von rheumatoider Arthritis; zur Vorbeugung der Alzheimer'schen Krankheit; Behandlung der Endometriose; Behandlung von Myomen; Behandlung von Myomen und der Endometriose in Kombination mit LHRH-Analoga; Behandlung hormonabhängiger Tumoren, z.B. des Mammacarcinoms, Behandlung prostatischer Erkrankungen.
Außerdem eignen sich die erfindungsgemäßen Verbindungen aufgrund ihres pharmakologischen Profils sowohl für die männliche als auch für die weibliche Kontrazeption.

Die Verbindungen können auch in Kombination mit dem natürlichen Vitamin D3 oder mit Calcitriol-Analoga für den Knochenaufbau oder als unterstützende Therapie zu Therapien, welche einen Knochenmassenverlust verursachen (beispielsweise eine Therapie mit Glucocorticoiden, Chemotherapie) eingesetzt werden.

Schließlich können die Verbindungen der allgemeinen Formel I in Verbindung mit Progesteronrezeptor-Antagonisten oder in Verbindung mit reinen Estrogenen verwendet werden, und zwar insbesondere zur Verwendung in der Hormonersatz-Therapie und zur Behandlung gynäkologischer Störungen und für die weibliche Fertilitätskontrolle.
Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der

Verabreichung kann die Menge der verabreichten Verbindung 0,01 - 10 mg / kg Körpergewicht, vorzugsweise 0,1 - 5 mg / kg Körpergewicht, je Tag betragen.
Beim Menschen entspricht dies einer Dosis von 0,8 bis 800 mg, vorzugsweise 8 bis 400 mg, täglich.

Eine Dosiseinheit enthält erfindungsgemäß 0,4 bis 400 mg einer oder mehrerer Verbindungen der allgemeinen Formel I.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfestoffe sowie sonstige übliche Träger- und Verdünnungsmittel verwendet werden können.
Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.
Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden. Schließlich können die Verbindungen auch mittels eines intrauterinen Systems (Spirale, IUD, Mirena® ) lokal verabreicht werden.

Die erfindungsgemäßen Verbindungen der allgemeinen Formel I werden wie in den Beispielen beschrieben hergestellt. Durch analoge Vorgehensweise unter Verwendung homologer Reagenzien zu den in den Beispielen beschriebenen Reagenzien lassen sich alle Verbindungen der allgemeinen Formel I erhalten.

Bei dem Verfahren zur Herstellung der Verbindungen der allgemeinen Formel I wird eine Verbindung der allgemeinen Formel II

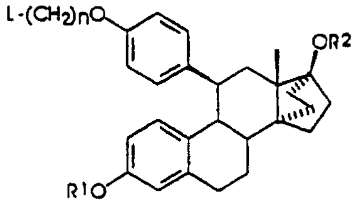

(II),

worin

$R^1$, $R^2$ und n die bereits in der allgemeinen Formel I angegebenen Bedeutungen haben und
L für eine Abgangsgruppe

steht,
wenn $R^3$ in der Verbindung der allgemeinen Formel I für eine Gruppierung $-(CH_2)_nNR^4R^5$ stehen soll,
mit einer Verbindung der allgemeinen Formel III

$$H\text{-}NR^4R^5 \quad (III)$$

worin $R^4$ und $R^5$ die in der allgemeinen Formel I angegebene Bedeutung haben, oder wenn $R^3$ in der Verbindung der allgemeinen Formel I für eine Gruppierung $-(CH_2)_nS(O)_mR^6$- stehen soll,
mit einer das Thiolat-Anion der allgemeinen Formel IV

$$^-SR6 \quad (IV)$$

liefernden Verbindung, worin $R^6$ die in der allgemeinen Formel I angegebene Bedeutung hat, umgesetzt, und die erhaltene Thioverbindung anschließend gegebenenfalls entweder zur Sulfinyl- (m = 1) oder Sulfonylverbindung (m = 2) oxidiert, oder 14,17-Ethano-11β-(4-hydroxyphenyl)-17-hydroxyestra-4,9-dien-3-on (V)

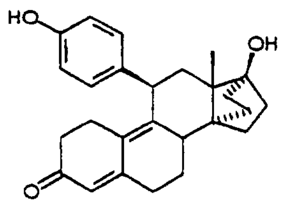

(V),

wenn $R^3$ in der Verbindung der allgemeinen Formel I für eine Gruppierung $-(CH_2)_nOCH_2CONR^4R^5$ stehen soll,
mit einer Verbindung der allgemeinen Formel VI

$$L\text{-}(CH_2)_nOCH_2CONR^4R^5 \quad (VI)$$

worin n, $R^4$ und $R^5$ die in der allgemeinen Formel I angegebene Bedeutung haben und L für eine Abgangsgruppe steht, zu einer Verbindung der allgemeinen Formel VII

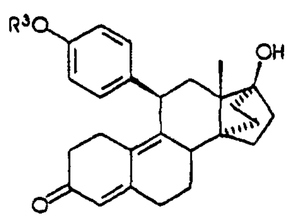

(VII),

umgesetzt und diese dann durch Aromatisierung des A-Ringes des Steroidgerüsts in eine Verbindung der allgemeinen Formel I, in der $R^1$ für ein Wasserstoffatom und $R^3$ für eine Gruppierung $-(CH_2)_nOCH_2CONR^4R^5$ steht, überführt wird.

Der Aufbau der Seitenkette $R^3$ kann dabei ganz analog wie bereits im US-Patent 5,149,696 -($R^3$ = $-(CH_2)_nOCH_2CONR^4R^5$) und in WO93/13123 ($R^3$ = $-(CH_2)_nS(O)_mR^6$) beschrieben erfolgen.
Im Falle, daß $R^3$ für $-(CH_2)_nNR^4R^5$ stehen soll, wird das entsprechende Iodid nach gängigen Verfahren mit einem Amin der Formel $HNR^4R^5$ umgesetzt.

Die bei der Herstellung der Verbindungen der allgemeinen Formel I durchlaufenen Zwischenverbindungen der allgemeinen Formel II

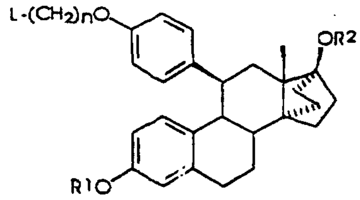

(II),

worin

$R^1$, $R^2$ und n die bereits in der allgemeinen Formel I angegebenen Bedeutungen haben und
L für eine Abgangsgruppe

steht,
gehören ebenfalls zum Gegenstand der vorliegenden Erfindung.

Vorzugsweise steht die Abgangsgruppe L für eine Hydroxygruppe, ein Chlor-, Brom- oder Iodatom oder für eine Tosylgruppe.

Auch mit anderen, dem Fachmann geläufigen, Abgangsgruppen L, wie beispielsweise der Mesylgruppe, läßt sich das erfindungsgemäße Verfahren durchführen.

Folgende Zwischenverbindungen sind besonders bevorzugt:

14,17-Ethano-11β-(4-[5-chlorpentyloxy]phenyl)-estra-1,3,5(10)-trien-3,17-diol
14,17-Ethano-11β-(4-[5-iodpentyloxy]phenyl)-estra-1,3,5(10)-trien-3,17-diol
14,17-Ethano-11β-(4-[6-hydroxyhexyloxy]phenyl)-estra-1,3,5(10)-trien-3,17-diol
3-Benzyloxy-14,17-ethano-11β-(4-[6-hydroxyhexyloxy]phenyl)-estra-1,3,5(10)-trien-17-ol
3-Benzyloxy-14,17-ethano-11β-(4-[6-tosyloxyhexyloxy]phenyl)-estra-1,3,5(10)-trien-17-ol.

Freie Hydroxygruppen $R^1$ und $R^2$ werden gegebenenfalls partiell in 3-Position oder vollständig in 3- und 17-Position mit einem Säurehalogenid oder -anhydrid der allgemeinen Formel V

$$R^{1'}X \text{ bzw. } R^{1'}_2O \qquad (V),$$

worin $R^{1'}$ eine $C_1$-$C_{12}$-Alkanoyl- oder Benzoylgruppe sowie X ein Chlor- oder Bromatom bedeuten, verestert oder partiell in 3-Position mit einem Alkylhalogenid der allgemeinen Formel VI

$$R^{1''}Y \qquad (VI),$$

worin $R^{1''}$ eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe sowie Y ein Chlor-, Brom- oder Iodatom bedeuten, verethert und gegebenenfalls in 17-Position mit einem Säurehalogenid- oder -anhydrid der allgemeinen Formel VII

$$R^{2'}X \text{ bzw. } R^{2'}_2O \qquad (VII),$$

worin $R^{2'}$ eine $C_1$-$C_{12}$-Alkanoylgruppe sowie X ein Chlor- oder Bromatom bedeuten, verestert.

Die nachfolgenden Beispiele dienen der näheren Erläuterung der Erfindung:

Beispiel 1

14α,17α-Ethano-11β-{4-[5-(pyrrolidin-1-yl)pentyloxy]phenyl}-1,3,5(10)-estratrien-3,17β-diol

a) 14,17-Ethano-11β-(4-[Dimethyl*tertiär*butylsilyloxy]phenyl)-17-hydroxyestra-4,9-dien-3-on

34 g Magnsiumspäne werden in 320 ml Tetrahydrofuran vorgelegt und mit einer Lösung von 360 g 4-Bromphenol*tertiär*butyldimethylsilylether in 560 ml Tetrahydrofuran innerhalb von 1,5 h versetzt. Nach 0,5 Stunden bei 80 °C Badtemperatur wird auf 0 °C abgekühlt, mit 6 g Kupfer(I)chlorid versetzt, 0,5 h bei 0 °C gerührt, eine Lösung von 42 g 3,3-(2,2-Dimethyltrimethylendioxy)-5α,10α-epoxy-14,17-ethanoestr-9(11)-en-17-ol [Scholz, S. et al. Liebigs Ann. Chem., (1989), S. 151 (13b)] in 560 ml Tetrahydrofuran zugetropft und 0,5 h bei 0 °C weitergerührt. Dann wird eine gesättigte Ammoniumchloridlösung zugegeben, 0,5 h bei 0 °C gerührt, mit Essigester verdünnt, mit Natriumchloridlösung gewaschen und im Vakuum zur Trockne eingeengt.

Man erhält 35 g rohes 11β-(4-[Dimethyl*tertiär*butylsilyloxy]phenyl)-3,3-(2,2-dimethyltrimethylendioxy)-14,17-ethanoestr-9-en-5α,17β-diol.

Das rohe 11β-(4-[Dimethyl*tertiär*butylsilyloxyaphenyl)-3,3-(2,2-dimethyltrimethylendioxy)-14,17-ethanoestr-9-en-5α, 17β-diol wird in 180 ml Tetrahydrofuran mit 200 ml Eisessig und 100 ml Wasser 3 h bei 50 °C Badtemperatur gerührt. Dann wird mit Essigester verdünnt, vier mal mit Natriumhydrogencarbonat und Kochsalzlösung gewaschen, über Natriumsulfat getrocknet, im Vakuum zur Trockne eingeengt und an Kieselgel mit Hexan/Essigester chromatographiert. Man erhält 29 g 14,17-Ethano-11β-(4-[Dimethyl*tertiär*butylsilyloxy]phenyl)-17-hydroxyestra-4,9-dien-3-on als Öl.

b) 14,17-Ethano-11β-(4-hydroxyphenyl)-17-hydroxyestra-4,9-dien-3-on

Eine Lösung von 15,1 g 14,17-Ethano-11β-(4-[dimethyl*tertiär*butylsilyloxy]phenyl)-17-hydroxyestra-4,9-dien-3-on in 65 ml Tetrahydrofuran wird mit 62 ml einer 1,1 M Tetrabutylammoniumfluorid-Lösung in Tetrahydrofuran 2 Stunden bei RT gerührt. Dann wird Ammoiniumchlorid-Lösung zugegeben, auf Wasser gegeben, dreimal mit Essigester extrahiert, neutralgewaschen, über Natriumsulfat getrocknet und i.Vak. eingeengt. Man erhält 13,3 g rohes 14,17-Ethano-11β-(4-hydroxyphenyl)-17-hydroxyestra-4,9-dien-3-on. Nach der Chromatographie an Kieselgel mit Hexan / Essigester erhält man 9,8 g reines 14,17-Ethano-11β-(4-hydroxyphenyl)-17-hydroxyestra-4,9-dien-3-on vom Schmelzpunkt 285-286 °C. $[\alpha]_D^{22}$ = +67,1° (c = 0.515% in Chloroform).

c) 14,17-Ethano-11β-(4-[5-chlorpentyloxy]phenyl)-17-hydroxyestra-4,9-dien-3-on

Eine Lösung von 13,3 g rohem in 145 ml Aceton und 40 ml einer 2 M wäßrigen Natriumhydroxid-Lösung wird bei Raumtemperatur mit 9,1 ml 1-Brom-5-chlorpentan in 30 ml Aceton versetzt und 3 Stunden bei 50 °C Badtemperatur gerührt. Dann wird mit Essigester verdünnt, mit 2 M Salzsäure, Natriumhydrogencarbonat- und Natriumchlorid-Lösung gewaschen, über Natriumsulfat getrocknet, i.Vak. eingeengt und an Kieselgel mit Hexan / Essigester chromatographiert. Man erhält 8 g reines 14,17-Ethano-11β-(4-[5-chlorpentyloxy]phenyl)-17-hydroxyestra-4,9-dien-3-on als Kristalle vom Schmelzpunkt 139 °C.

d) 14,17-Ethano-11β-(4-[5-chlorpentyloxy]phenyl)-estra-1,3,5(10)-trien-3,17-diol

Eine Lösung von 8 g 14,17-Ethano-11β-(4-[5-chlorpentyloxy]phenyl)-17-hydroxyestra-4,9-dien-3-on in 100 ml abs. Ethanol wird mit 1 g Palladium auf Aktivkohle 4,5 Stunden bei 110 °C Badtemperatur gerührt. Dann wird über Celite filtriert, mit Essigester nachgewaschen und i.Vak. eingeengt. Man erhält 8 g rohes 14,17-Ethano-11β-(4-[5-chlorpentyloxy]phenyl)-estra-1,3,5(10)-trien-3,17-diol. Wird an Kieselgel mit Hexan / Essigester chromatographiertals erhält man Kristalle vom Schmelzpunkt 215 °C.

e) 14,17-Ethano-11β-(4-[5-iodpentyloxy]phenyl)-estra-1,3,5(10)-trien-3,17-diol

Eine Lösung von 8 g rohem 14,17-Ethano-11β-(4-[5-Chlorpentyloxy]phenyl)-estra-1,3,5(10)-trien-3,17-diol in 150 ml Ethylmethylketon wird mit 6,1 g Natriumiodid 24 Stunden bei 80 °C Badtemperatur gerührt. Anschließend wird mit Essigester verdünnt, mit Wasser und Natriumchlorid-Lösung gewaschen, über Natriumsulfat getrocknet, i.Vak. eingeengt und an Kieselgel mit Hexan / Essigester chromatographiert. Man erhält 5,2 g 14,17-Ethano-11β-(4-[5-iodpentyloxy]phenyl)-estra-1,3,5(10)-trien-3,17-diol.

f) 14α,17α-Ethano-11β-{4-[5-(pyrrolidin-1-yl)pentyloxy]phenyl}-1,3,5(10)-estratrien-3,17β-diol

Eine Lösung von 500 mg 14,17-Ethano-11β-(4-[5-iodpentyloxy]phenyl)-estra-1,3,5(10)-trien-3,17-diol in 5 ml Dimethylformamid wird mit 0,3 ml Pyrrolidin 3,5 Stunden bei 100 °C Badtemperatur gerührt. Zur Aufarbeitung wird auf Wasser gegeben, mit Essigester extrahiert, mit 0,1 M Salzsäure gewaschen, neutralgewaschen, über Natriumsulfat getrocknet, i.Vak. eingeengt und an Kieselgel mit Hexan / Essigester chromatographiert. Man erhält 110 mg reines 14α,17α-Ethano-11β-{4-[5-(pyrrolidin-1-yl)pentyloxy)phenyl}-1,3,5(10)-estratrien-3,17β-diol als Kristalle vom Schmelzpunkt 208-210 °C. $[\alpha]_D^{22}$ = -143.5° (c = 0.510% in Chloroform).

Beispiel 2

14α,17α-Ethano-11β-{4-[5-(pentylthio)pentyloxy]phenyl}-1,3,5(10)-estratrien-3,17β-diol

Zu einer Lösung von 1 g 14,17-Ethano-11β-(4-[5-iodpentyloxy]phenyl)-estra-1,3,5(10)-trien-3,17-diol in 20 ml Dimethylformamid tropft man eine Lösung von 292 mg Pentylthioacetat in 2 ml Methanol und rührt 1 Stunde bei Raumtemperatur. Dann wird auf 1 M Salzsäure gegeben, dreimal mit Essigester extrahiert, neutralgewaschen, über Natriumsulfat getrocknet, i.Vak. eingeengt und an Kieselgel mit Hexan / Essigester chromatographiert. Man erhält 850 mg reines 14α,17α-Ethano-11β-{4-[5-(pentylthio)pentyloxy]phenyl}-1,3,5(10)-estratrien-3,17β-diol als Kristalle vom Schmelzpunkt 73-75 °C. $[\alpha]_D^{22}$ = -26.0° (c = 0.550% in Chloroform)

Beispiel 3

14α,17α-Ethano-11β-{4-[5-(pentansulfinyl)pentyloxy]phenyl}-1,3,5(10)-estratrien-3,17β-diol

Eine Lösung von 400 mg 14α,17α-Ethano-11β-{4-[5-(pentylthio)pentyloxy]phenyl}-1,3,5(10)-estratrien-3,17β-diol in 15 ml Methanol und 0,65 ml Wasser wird mit 176 mg Natriumperiodat 4,5 Stunden bei Raumtemperatur gerührt. Zur Aufarbeitung wird auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 393 mg reines 14α,17α-Ethano-11β-{4-[5-(pentansulfinyl)pentyloxy]phenyl}-1,3,5(10)-estratrien-3,17β-diol als Kristalle vom Schmelzpunkt 123-128 °C. $[\alpha]_D^{22}$ = -53.2° (c = 0.550 % in Chloroform)

Beispiel 4

14α,17α-Ethano-11β-{4-[5-(pentansulfonyl)pentyloxy]phenyl}-1,3,5(10)-estratrien-3,17β-diol

Eine Lösung von 400 mg 14α,17α-Ethano-11β-{4-[5-(pentylthio)pentyloxy]phenyl}-1,3,5(10)-estratrien-3,17β-diol in 10 ml 1,2-Dichlorethan und 10 ml *tertiär*-Butanol wird mit 507 mg m-Chlorperbenzoesäure portionsweise versetzt und 2 Stunden gerührt. Dann wir mit gesättigter Natriumhydrogensulfit-Lösung versetzt, auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 231 mg reines 14α,17α-Ethano-11β-{4-[5-(pentansulfonyl) pentyloxy]phenyl}-1,3,5(10)-estratrien-3,17β-diol als Kristalle vom Schmelzpunkt 107-108 °C. $[\alpha]_D^{22}$ = -38.2° (c = 0.5 % in Chloroform)

Beispiel 5

14α,17α-Ethano-11β-{4-[5-(4,4,5,5,5-pentafluorpentylsulfinyl)pentyloxy]phenyl}-1,3,5(10)-estratrien-3,17β-diol

a) 14α,17α-Ethano-11β-{4-[5-(4,4,5,5,5-pentafluorpentylthio)pentyloxy]phenyl}-1,3,5(10)-estratrien-3,17β-diol

Eine Lösung von 500 mg 14,17-Ethano-11β-(4-[5-iodpentyloxy]phenyl)-estra-1,3,5(10)-trien-3,17-diol in 10 ml Dimethylformamid wird bei Raumtemperatur tropfenweise mit einer Lösung von 227,6 mg 4,4,5,5,5-Pentafluorpentyl-1-thioacetat in 1 ml Methanol und 0,19 ml 30%iger Natriummethylat-Lösung versetzt und 2 Stunden gerührt. Dann wird auf 1 M Salzsäure gegeben, dreimal mit Essigester extrahiert, neutralgewaschen, über Natriumsulfat getrocknet, i.Vak. eingeengt und an Kieselgel mit Hexan / Essigester chromatographiert. Man erhält 500 mg 14α,17α-Ethano-11β-{4-[5-(4,4,5,5,5-pentafluorpentylthio)pentyloxy]phenyl}-1,3,5(10)-estratrien-3,17β-diol.

b) 14α,17α-Ethano-11β-{4-[5-(4,4,5,5,5-pentafluorpentylsulfinyl)pentyloxy]phenyl}-1,3,5(10)-estratrien-3,17β-diol

Eine Lösung von 500 mg 14α,17α-Ethano-11β-{4-[5-(4,4,5,5,5-pentafluorpentyl-thio)pentyloxy]phenyl}-1,3,5(10)-estratrien-3,17β-diol in 15 ml Methanol und 0,65 ml Wasser wird mit 200 mg Natriumperiodat 6 Stunden bei Raumtemperatur gerührt. Zur Aufarbeitung wird auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 390 mg reines 14α,17α-Ethano-11β-{4-[5-(4,4,5,5,5-pentafluorpentylsulfinyl)pentyloxy]phenyl}-1,3,5(10)-estratrien-3,17β-diol als Kristalle vom Schmelzpunkt 110-115 °C. $[\alpha]_D^{22}$ = -40.3° (c = 0.505 % in Chloroform)

Beispiel 6

14α,17α-Ethano-11β-{4-[5-(2-pyridinmethylsulfonyl)pentyloxy]phenyl}-1,3,5(10)-estratrien-3,17β-diol

Eine Lösung von 400 mg 14α,17α-Ethano-11β-{4-[5-(2-pyridinmethylsulfinyl)-pentyloxy]phenyl}-1,3,5(10)-estratrien-3,17β-diol (Beispiel 10) in 7,5 ml 1,2-Dichlorethan und 7,5 ml *tertiär*-Butanol wird mit 200 mg *m*-Chlorperbenzoesäure portionsweise versetzt und 2 Stunden gerührt. Dann wird mit gesättigter Natriumhydrogensulfit-Lösung versetzt, auf Wasser gegeben, dreimal mit Essigester extrahiert, neutralgewaschen, über Natriumsulfat getrocknet, i. Vak. eingeengt und an Kieselgel mit Dichlormethan / Aceton chromatographiert. Man erhält 184,4 mg reines 14α, 17α-Ethano-11β-{4-[5-(2-pyridinmethylsulfonyl)pentyloxy]phenyl}-1,3,5(10)-estratrien-3,17β-diol als Kristalle vom Schmelzpunkt 98-100 °C. $[\alpha]_D^{22}$ = -39.8° (c = 0.540 % in Chloroform)

Beispiel 7

14α,17α-Ethano-11β-{4-[6-(4,4,5,5,5-pentafluorpentylthio)hexyloxy]-phenyl}-1,3,5(10)-estratrien-3,17β-diol

a) 14,17-Ethano-11β-(4-[6-dimethyl*tertiär*butylsilyloxyhexyloxy]phenyl)-17-hydroxyestra-4,9-dien-3-on

Eine Lösung von 15 g 14,17-Ethano-11β-(4-hydroxyphenyl)-17-hydroxyestra-4,9-dien-3-on in 150 ml Aceton und 40 ml 2 M wäßriger Natriumhydroxid-Lösung wird bei Raumtemperatur mit einer Lösung von 30 g 1-Brom-6-dimethyl*tertiär*butylsilyloxyhexan in 30 ml Aceton tropfenweise versetzt und anschließend 6 Stunden bei 50 °C gerührt. Zur Aufarbeitung wird Essigester zugegeben, mit 2 M Salzsäure extrahiert, mit Natriumhydrogencarbonat- und Kochsalzlösung gewaschen, über Natriumsulfat getrocknet und i.Vak. eingeengt. Man erhält 28,9 g Rohprodukt. Nach Chromatographie an Kieselgel mit Hexan / Essigester erhält man 13 g reines 14,17-Ethano-11β-(4-[6-dimethyl*tertiär*butylsilyloxyhexyloxy]phenyl)-17-hydroxyestra-4,9-dien-3-on als Kristalle vom Schmelzpunkt 122-124 °C. $[\alpha]_D^{22}$ = +121,2° (c = 0.550 % in Chloroform)

b) 14,17-Ethano-11β-(4-[6-hydroxyhexyloxy]phenyl)-estra-1,3,5(10)-trien-3,17-diol

Eine Lösung von 11,1 g 14,17-Ethano-11β-(4-[6-dimethyl*tertiär*butylsilyloxyhexyloxy]phenyl)-17-hydroxyestra-4,9-dien-3-on in 111 ml abs. Ethanol wird mit 1,11 g Palladium auf Aktivkohle 4 Stunden bei 100 °C Badtemperatur gerührt. Dann wird über Celite filtriert, mit Essigester nachgewaschen und i.Vak. eingeengt. Man erhält 11 g rohes 14,17-Ethano-11β-(4-[6-dimethyl*tertiär*butylsilyloxyhexyloxy]phenyl)-estra-1,3,5(10)-trien-3,17-diol.
Einen Lösung von 11 g des rohen 14,17-Ethano-11β-(4-[6-dimethyl*tertiär*butylsilyloxyhexyloxy]phenyl)-estra-1,3,5(10)-trien-3,17-diols in 55 ml Tetrahydrofuran wird mit 62 ml Eisessig und 31 ml Wasser 2,5 Stunden bei 50 °C Badtemperatur gerührt. Anschließend wird mit Essigester verdünnt, mit Natriumhydrogencarbonat- und Natriumchlorid-Lösung gewaschen, über Natriumsulfat getrocknet, i.Vak. eingeengt und an Kieselgel mit Dichlormethan / Aceton chromatographiert. Man erhält 8 g 14,17-Ethano-11β-(4-[6-hydroxyhexyloxy]phenyl)-estra-1,3,5(10)-trien-3,17-diol als Kristalle vom Schmelzpunkt 116-118 °C. $[\alpha]_D^{22}$ = -18,5° (c = 0,540 % in Chloroform)

c) 3-Benzyloxy-14,17-ethano-11β-(4-[6-hydroxyhexyloxy]phenyl)-estra-1,3,5(10)-trien-17-ol

Eine Lösung von 2,5 g 14,17-Ethano-11β-(4-[6-hydroxyhexyloxy]phenyl)-estra-1,3,5(10)-trien-3,17-diol in 30 ml Acetonitril wird mit 774 mg Kaliumcarbonat und 0,67 ml Benzylbromid 3 Stunden bei 100 °C Badtemperatur gerührt. Dann wird bei 50 °C Badtemperatur i.Vak. eingeengt, auf Wasser gegeben, dreimal mit Dichlormethan extrahiert, mit Kochsalz-Lösung gewaschen, über Natriumsulfat getrocknet, i.Vak. eingeengt und an Kieselgel mit Dichlormethan / Aceton chromatographiert. Man erhält 2,1 g reines 3-Benzyloxy-14,17-ethano-11β-(4-[6-hydroxyhexyloxy]phenyl)-estra-1,3,5(10)-trien-17-ol als Kristalle vom Schmelzpunkt 142-144 °C. $[\alpha]_D^{22}$ = -30,1° (c = 0.520 % in Chloroform)

d) 3-Benzyloxy-14,17-ethano-11β-(4-[6-tosyloxyhexyloxy]phenyl)-estra-1,3,5(10)-trien-17-ol

Eine Lösung von 850 mg 3-Benzyloxy-14,17-ethano-11β-(4-[6-hydroxyhexyloxy]phenyl)-estra-1,3,5(10)-trien-17-ol in 10 ml Pyridin wird mit 500 mg Toluolsulfonsäureanhydrid 3,5 Stunden bei Raumtemperatur gerührt. Zur Aufarbeitung wird Essigester zugegeben, mit 2 M Salzsäure extrahiert, mit Natriumhydrogencarbonat- und Kochsalzlösung gewaschen, über Natriumsulfat getrocknet und i.Vak. eingeengt. Man erhält 1 g Rohprodukt. Nach Chromatographie an Kieselgel mit Hexan / Essigester erhält man 670 mg reines 3-Benzyloxy-14,17-ethano-11β-(4-[6-tosyloxyhexyloxy]phenyl)-estra-1,3,5(10)-trien-17-ol als Kristalle vom Schmelzpunkt 70-71 °C. $[\alpha]_D^{22}$ = -29,3° (c = 0,525 % in Chloroform)

e) 3-Benzyloxy-14α,17α-ethano-11β-{4-[6-(4,4,5,5,5-pentafluorpentylthio)hexyloxy]-phenyl}-1,3,5(10)-estratrien-17β-ol

Eine Lösung von 600 mg 3-Benzyloxy-14,17-ethano-11β-(4-[6-tosyloxyhexyloxy]phenyl)-estra-1,3,5(10)-trien-17-ol 10 ml Dimethylformamid wird bei Raumtemperatur tropfenweise mit einer Lösung von 227,6 mg 4,4,5,5,5-Pentafluorpentyl-1-thioacetat in 1 ml Methanol und 0,19 ml 30%iger Natriummethylat-Lösung versetzt und 2 Stunden gerührt. Dann wird auf 1 M Salzsäure gegeben, dreimal mit Essigester extrahiert, neutralgewaschen, über Natriumsulfat getrocknet, i.Vak. eingeengt und an Kieselgel mit Hexan / Essigester chromatographiert. Man erhält 630 mg als Öl. $[\alpha]_D^{22}$ = -26,4° (c = 0.525 % in Chloroform)

f) 14α,17α-Ethano-11β-{4-[6-(4,4,5,5,5-pentafluorpentylthio)hexyloxy]-phenyl}-1,3,5(10)-estratrien-3,17β-diol

Eine Lösung von 600 mg 3-Benzyloxy-14α,17α-ethano-11β-{4-[6-(4,4,5,5,5-pentafluorpentylthio)hexyloxy]-phenyl}-1,3,5(10)-estratrien-17β-ol in 10 ml Dichlormethan wird bei 0 °C mit 288 mg N,N-Dimethylanilin langsam versetzt und nach Zugabe von 420 mg Aluminiumtrichlorid 4 Stunden bei 0 °C gerührt. Dann wird bei 0 °C mit Wasser versetzt, mit 1 M Schwefelsäure angesäuert, dreimal mit Essigester extrahiert, neutralgewaschen, über Natriumsulfat getrocknet i.Vak. eingeengt und an Kieselgel mit Hexan / Essigester chromatographiert. Man erhält 430,3 mg 14α, 17α-Ethano-11β-{4-[6-(4,4,5,5,5-pentafluorpentylthio)hexyloxy]-phenyl}-1,3,5(10)-estratrien-3,17β-diol als Kristalle 63-64 °C. $[\alpha]_D^{22}$ = -32,6° (c = 0.515 % in Chloroform)

Beispiel 8

14α,17α-Ethano-11β-{4-[6-(4,4,5,5,5-pentafluorpentyl)sulfinyl]hexyloxy}phenyl-1,3,5(10)-estratrien-3,17β-diol

Eine Lösung von 180 mg 14α,17α-Ethano-11β-{4-[6-(4,4,5,5,5-pentafluorpentyl-thio)hexyloxy]-phenyl}-1,3,5(10)-estratrien-3,17β-diol in 5,4 ml Methanol und 0,23 ml Wasser wird mit 72 mg Natriumperiodat 5 Stunden bei Raumtemperatur gerührt. Zur Aufarbeitung wird auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 134 mg reines 14α,17α-Ethano-11β-{4-[6-(4,4,5,5,5-pentafluorpentyl)sulfinyl]hexyloxy}phenyl-1,3,5(10)-estratrien-3,17β-diol als Kristalle vom Schmelzpunkt 118-120 °C. $[\alpha]_D^{22}$ = -38,3° (c = 0.530 % in Chloroform)

Beispiel 9

14α,17α-Ethano-11β-{4-[6-(4,4,5,5,5-pentafluorpentyl)sulfonyl]hexyloxy}phenyl-1,3,5(10)-estratrien-3,17β-diol

Eine Lösung von 200 mg 14α,17α-Ethano-11β-{4-[6-(4,4,5,5,5-pentafluorpentyl-thio)hexyloxyl-phenyl}-1,3,5(10)-estratrien-3,17β-diol in 3,5 ml 1,2-Dichlorethan und 3,5 ml *tertiär*-Butanol wird mit 100 mg *m*-Chlorperbenzoesäure portionsweise versetzt und 1 Stunden gerührt. Dann wird auf gesättigte Natriumhydrogensulfit-Lösung gegeben, dreimal mit Essigester extrahiert, neutralgewaschen, über Natriumsulfat getrocknet, i.Vak. eingeengt und an Kieselgel mit Hexan / Essigester chromatographiert. Man erhält 154 mg reines 14α,17α-Ethano-11β-{4-[6-(4,4,5,5,5-pentafluorpentyl)sulfonyl]hexyloxy)phenyl-1,3,5(10)-estratrien-3,17β-diol als Kristalle vom Schmelzpunkt 93-95 °C. $[\alpha]_D^{22}$ = -38,0° (c = 0,515 % in Chloroform)

Beispiel 10

14α,17α-Ethano-11β-{4-[5-(2-pyridinmethylsulfinyl)pentyloxy]phenyl}-1,3,5(10)-estratrien-3,17β-diol

a) 14α,17α-Ethano-11β-{4-[5-(2-pyridinmethylthio)pentyloxy]phenyl}-1,3,5(10)-estratrien-3,17β-diol

Eine Lösung von 1,94 g 14,17-Ethano-11β-(4-[5-iodpentyloxy]phenyl)-estra-1,3,5(10)-trien-3,17-diol in 40 ml Methanol wird zu einer vorbereiteten Lösung von 12,5 g einer 10%igen ethanolischen 2-Mercaptomethylpyridin-Lösung und 1,7 ml 30%iger Natriummethylat-Lösung getropft, mit 600 mg Natriumiodid versetzt und 1 Stunde bei 80 °C Badtemperatur gerührt. Zur Aufarbeitung wird auf Wasser gegeben, dreimal mit Essigester extrahiert, mit gesättigter Kochsalzlösung gewaschen, über Natriumsulfat getrocknet, i.Vak. eingeengt und an Kieselgel mit Dichlormethan / Aceton chromatographiert. Man erhält 14α,17α-Ethano-11β-{4-[5-(2-pyridinmethylthio)pentyloxy]phenyl}-1,3,5(10)-estratrien-3,17β-diol als Kristalle vom Schmelzpunkt 93-95 °C. $[\alpha]_D^{22}$ = -44.1° (c = 0.525 % in Chloroform)

b) 14α,17α-Ethano-11β-{4-[5-(2-pyridinmethylsulfinyl)pentyloxy]phenyl}-1,3,5(10)-estratrien-3,17β-diol

Eine Lösung von 1,3 g 14α,17α-Ethano-11β-{4-[5-(2-pyridinmethylthio)pentyloxy]phenyl}-1,3,5(10)-estratrien-3,17β-diol in 50 ml Methanol und 2,1 ml Wasser wird mit 570 mg Natriumperiodat 3,5 Stunden bei Raumtemperatur gerührt. Zur Aufarbeitung wird auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 1,1 g reines 14α,17α-Ethano-11β-{4-[5-(2-pyridinmethylsulfinyl)pentyloxy]phenyl}-1,3,5(10)-estratrien-3,17β-diol als Kristalle vom Schmelzpunkt 118-120 °C. $[\alpha]_D^{22}$ = -52.6° (c = 0.505 % in Chloroform)

Beispiel 11

14α,17α-Ethano-11β-{4-[5-(4,4,5,5,5-pentafluorpentyl)sulfonyl]pentyloxy}phenyl-1,3,5(10)-estratrien-3,17β-diol

Eine Lösung von 1,85 g 14α,17α-Ethano-11β-{4-[5-(4,4,5,5,5-pentafluorpentyl-thio)pentyloxy]phenyl}-1,3,5(10)-estratrien-3,17β-diol in 72 ml 1,2-Dichlorethan und 72 ml *tertiär*-Butanol wird mit 2,6 g *m*-Chlorperbenzoesäure portionsweise versetzt und 1 Stunden bei Raumtemperatur gerührt. Dann wird mit Dichlormethan verdünnt. mit gesättigter Natriumhydrogensulfit-, Natriumhydrogencarbonat-und Kochsalz-Lösung gewaschen, über Natriumsulfat getrocknet, i.Vak. eingeengt und an Kieselgel mit Hexan / Essigester chromatographiert. Man erhält 800 mg reines 14α, 17α-Ethano-11β-{4-[5-(4,4,5,5,5-pentafluorpentyl)sulfonyl]pentyloxy}phenyl-1,3,5(10)-estratrien-3,17β-diol. $[\alpha]_D^{22}$ = -36,6° (c = 0,5 % in Chloroform)

Beispiel 12

2-(6-[4-{14,17-Ethano-3,17-dihydroxyestra-1,3,5(10)-trien-11β-yl}-phenoxy]-hexyloxy)essigsäure-(N-butyl-N-methyl)-amid

a) 2-(6-[4-{14,17-Ethano-17-hydroxyestra-3-oxo-4,9-dien-11β-yl}phenoxy]hexyloxy) essigsäure-(N-butyl-N-methyl)-amid

Eine Lösung von 2,5 g 14,17-Ethano-11β-(4-hydroxyphenyl)-17-hydroxyestra-4,9-dien-3-on in 40 ml Methanol und 10 ml 2 M wäßriger Natriumhydroxid-Lösung wird mit 3,8 g 2-(6-Bromhexyloxy)essigsäure-(N-butyl-N-methyl)-amid in 6 ml Aceton tropfenweise versetzt und 4,5 Stunden bei 50 °C Badtemperatur gerührt. Dann wird mit Essigester verdünnt, mit 1 M Salzsäure gewaschen, mit Natriumhydrogencarbonat- und Kochsalz-Lösung gewaschen, über Natriumsulfat getrocknet, i.Vak. eingeengt und an Kieselgel mit Hexan / Essigester chromatographiert. Man erhält 3,1 g reines 2-(6-[4-{14,17-Ethano-17-hydroxyestra-3-oxo-4,9-dien-11β-yl}phenoxy]hexyloxy)essigsäure-(N-butyl-N-methyl)-amid als Schaum. $[\alpha]_D^{22}$ = +126,6° (c = 0.525 % in Chloroform)

b) 2-(6-(4-{14,17-Ethano-3,17-dihydroxyestra-1,3,5(10)-trien-11β-yl}-phenoxy]-hexyloxy)essigsäure-(N-butyl-N-methyl)-amid

Eine Lösung von 500 mg 2-(6-[4-{14,17-Ethano-17-hydroxyestra-3-oxo-4,9-dien-11β-yl}phenoxy]hexyloxy) essigsäure-(N-butyl-N-methyl)-amid in 10 ml Methanol wird mit 0,5 g Palladiumhydroxid auf Magnesiumoxid 0,5 Stunden bei 80 °C Badtemperatur gerührt. Dann wird über Celite filtriert, mit Essigester nachgewaschen, i.Vak. eingeengt und an Kieselgel mit Hexan / Essigester chromatographiert. Man erhält 130 mg reines 2-(6-[4-{14,17-Ethano-3,17-dihydroxyestra-1,3,5(10)-trien-11β-yl}-phenoxy]-hexyloxy)essigsäure-(N-butyl-N-methyl)-amid als Kristalle vom Schmelzpunkt 94-95 °C. $[\alpha]_D^{22}$ = -32,2° (c = 0,500 % in Chloroform)

Tabelle 1

| Antiestrogene/estrogene Wirkung in vitro | | | |
|---|---|---|---|
| | Antagonismus | | Agonismus HELA (rPR-TK) [% von Estradiol] |
| | HELA | MVLN | |
| | $IC_{50}$[nM] | | |
| Verbindung | | | |
| A | 0.4 | -- | 16 |
| B | 0.3 | 0.4 | 50 |

Tabelle 1 (fortgesetzt)

| Antiestrogene/estrogene Wirkung in vitro | | | |
|---|---|---|---|
| | Antagonismus | | Agonismus HELA (rPR-TK) [% von Estradiol] |
| | HELA | MVLN | |
| | $IC_{50}$[nM] | | |
| Verbindung | | | |
| V | 0.14 | 1.0 | 37 |

Tabelle 2

| Uteruswachstumstest: | | | |
|---|---|---|---|
| Verbindung | Applikationsart | Uterusgewichte % E2 | Epithelhöhe % E2 |
| 0.01 mg/kg A | s.c. | 2 | 6 |
| 0.1 mg/kg A | s.c. | 0 | -16 |
| 1 mg/kg A | s.c. | -7 | -8 |
| 10 mg/kg A | s.c. | -15 | -22 |
| 0.01 mg/kg B | s.c. | 3 | 4 |
| 0.1 mg/kg B | s.c. | 31 | 19 |
| 1 mg/kg B | s.c. | 14 | 18 |
| 10 mg/kg B | s.c. | 6 | 13 |
| 0.03 mg/kg V | s.c. | 28 | 18 |
| 0.3 mg/kg V | s.c. | 32 | 33 |
| 3 mg/kg V | s.c. | 19 | 27 |

Tabelle 3

| Antiuteruswachstumstest: | | |
|---|---|---|
| Verbindung | Applikationsart | Uterusgewichte % Hemmung |
| 0.1 mg/kg A | s.c. | 33 |
| 1 mg/kg A | s.c. | 88 |
| 10 mg/kg A | s.c. | 121 |
| 0.1 mg/kg B | s.c. | 32 |
| 1 mg/kg B | s.c. | 83 |
| 10 mg/kg B | s.c. | 100 |
| 0.3 mg/kg V | s.c. | 57 |

Knochendichtebestimmungen:

Tabelle 4

| Verbindung A | | | | | | |
|---|---|---|---|---|---|---|
| **Gruppe** | **Testsubstanz** | **Dosis/Tier s. c.** | **Knochendichte Femur - mg Ca/ ccm** | **% Wirkung von E2** | **TB/TV (Tibia) in %** | **% Wirkung von E2** |
| 1 | ovx- Kontrolle | - | 286,1 | | 16,1 | |
| 2 | 17 β Estradiol | 0.3 µg | 361,7 | 100 | 34.0 | |
| 3 | Verbindung A | 0,1 µg | 296,4 | 14 | 17.2 | 4,1 |
| 4 | Verbindung A | 1 µg | 309,6 | 31 | 18,9 | 17 |
| 5 | Verbindung A | 10 µg | 331,3 | 60 | 22,5 | 35 |
| 6 | Verbindung A | 100 µg | 324,1 | 50 | 27,0 | 62 |
| 7 | sham | - | | | 32.2 | |

Tabelle 5

| Verbindung A | | | | |
|---|---|---|---|---|
| **Gruppe** | **Testsubstanz** | **Dosis/Tier s.c.** | **Knochendichte Tibia-mg Ca/ccm** | **% Wirkung von E2** |
| 1 | ovx- Kontrolle | -- | 299,4 | |
| 2 | 17 β Estradiol | 0,3 µg | 375,2 | 100 |
| 3 | Verbindung A | 100 µg | 361.8 | 82 |
| 4 | Verbindung A | 500 µg | 336.3 | 49 |
| 5 | Verbindung A | 2000 µg | 348,8 | 65 |

Tabelle 6

| Verbindung B | | | | |
|---|---|---|---|---|
| **Gruppe** | **Testsubstanz** | **Dosis/Tier s.c.** | **TB/TV (Tibia) in %** | **% Wirkung von E2** |
| 1 | ovx- Kontrolle | -- | 13,0 | |
| 2 | 17 β Estradiol | 0,3µg | 26,0 | 100 |
| 3 | Verbindung B | 1 µg | 19,6 | 52 |
| 4 | Verbindung B | 10 µg | 18,9 | 47 |
| 5 | Verbindung B | 100 µg | 18,6 | 45 |

Tabelle 7

| Verbindung V | | | | |
|---|---|---|---|---|
| **Gruppe** | **Testsubstanz** | **Dosis/Tier s.c.** | **Knochendichte Tibia- mg Ca/ccm** | **% Wirkung von E2** |
| 1 | ovx- Kontrolle | -- | 293,7 | |
| 2 | 17β Estradiol | 0,3 µg | 402,9 | 100 |
| 3 | Verbindung V | 10 µg | 337,3 | 40 |
| 4 | Verbindung V | 100 µg | 331,2 | 34,4 |

Tabelle 7 (fortgesetzt)

| Verbindung V | | | | |
|---|---|---|---|---|
| **Gruppe** | **Testsubstanz** | **Dosis/Tier s.c.** | **Knochendichte Tibia- mg Ca/ccm** | **% Wirkung von E2** |
| 5 | Verbindung V | 250 µg | 374,3 | 74 |

**Patentansprüche**

**1.** 11β-arylsubstituierte 14,17-Ethanoestratriene der allgemeinen Formel I

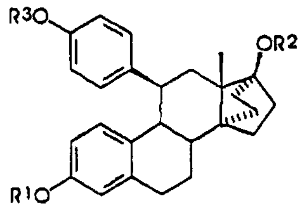

(I),

worin

$R^1$ für ein Wasserstoffatom, eine $C_1$-$C_{12}$-Alkanoyl-, eine Benzoyl-, eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe, die alle gegebenenfalls substituiert sein können,
$R^2$ für ein Wasserstoffatom oder eine gegebenenfalls substituierte $C_1$-$C_{12}$-Alkanoylgruppe und
$R^3$ für eine Gruppierung
$-(CH_2)_nNR^4R^5$ oder $-(CH_2)_nOCH_2CONR^4R^5$ oder $-(CH_2)_nS(O)_mR^6$

stehen,
worin

n 4, 5, 6 oder 7 ist,
m 0, 1 oder 2 ist,
$R^4$ oder $R^5$ unabhängig voneinander ein Wasserstoffatom oder eine geradkettige oder verzweigte, gegebenenfalls substituierte $C_1$-$C_8$-Alkylgruppe, die auch teilweise oder vollständig fluoriert sein kann, oder
$R^4$ und $R^5$ gemeinsam eine Methylen- bis Pentamethylengruppe $-(CH_2)_p-$ (p = 1 - 5) bedeuten und
$R^6$ die Bedeutung von $R^4$ oder $R^5$ hat oder ein gegebenenfalls substituierter Aryl- oder Arylalkylrest ist, wobei der Alkylrest darin bis zu 6 Kohlenstoffatome haben kann und der Arylrest, alleine oder im Arylalkylrest, ein 5- oder 6gliedriger monocyclischer Ring oder ein kondensiertes, 8 bis 10gliedriges Ringsystem ist und der Arylrest ein- oder mehrere Heteroatome ausgewählt unter Sauerstoff, Stickstoff und Schwefel aufweisen kann.

**2.** 11β-arylsubstituierte 14,17-Ethanoestratriene nach Anspruch 1, worin $R^1$ ein Wasserstoffatom ist.

**3.** 11β-arylsubstituierte 14,17-Ethanoestratriene nach Anspruch 1, worin $R^2$ ein Wasserstoffatom ist.

**4.** 11β-arylsubstituierte 14,17-Ethanoestratriene nach Anspruch 1, worin $R^3$ für eine Gruppierung $-(CH_2)_nNR^4R^5$ steht.

**5.** 11β-arylsubstituierte 14,17-Ethanoestratriene nach Anspruch 1, worin $R^3$ für eine Gruppierung $-(CH_2)_nOCH_2CONR^4R^5$ steht.

**6.** 11β-arylsubstituierte 14,17-Ethanoestratriene nach Anspruch 1, worin $R^3$ für eine Gruppierung $-(CH_2)_nS(O)_mR^6$ steht.

**7.** 11β-arylsubstituierte 14,17-Ethanoestratriene nach Anspruch 6, worin $R^6$ für eine n-Pentyl- oder $-CH_2$-(2-Pyridin)-,

$-CH_2$-(3-Pyridin)- oder $-CH_2$-(4-Pyridin)gruppe steht.

**8.** 11β-arylsubstituierte 14,17-Ethanoestratriene nach Anspruch 6, worin $R^6$ für eine 4,4,5,5,5-Pentafluorpentylgruppe steht.

**9.** 11β-arylsubstituierte 14,17-Ethanoestratriene nach Anspruch 1, worin $R^3$ für einen der folgenden Substituenten steht:

$$-(CH_2)_5S(CH_2)_4CH_3$$

$$-(CH_2)_5SO(CH_2)_4CH_3$$

$$-(CH_2)_5SO_2(CH_2)_4CH_3$$

$$-(CH_2)_5SO(CH_2)_3CF_2CF_3$$

$$-(CH_2)_5SO_2\text{-}CH_2\text{-(2-Pyridyl)}$$

$$-(CH_2)_5SO_2\text{-}CH_2\text{-(3-Pyridyl)}$$

$$-(CH_2)_5SO_2\text{-}CH_2\text{-(4-Pyridyl)}$$

$$-(CH_2)_6\text{-O-}CH_2C(O)N(CH_3)C_4H_9$$

$$-(CH_2)_5SO_2(CH_2)_3CF_2CF_3$$

$$-(CH_2)_5SO\text{-}CH_2\text{-(2-Pyridyl)}$$

$$-(CH_2)_5SO\text{-}CH_2\text{-(3-Pyridyl)}$$

$$-(CH_2)_5SO\text{-}CH_2\text{-(4-Pyridyl)}$$

$$-(CH_2)_5\text{-Pyrrolidinyl}$$

$$-(CH_2)_6S(CH_2)_3CF_2CF_3$$

$$-(CH_2)_6SO(CH_2)_3CF_2CF_3$$

$$-(CH_2)_6SO_2(CH_2)_3CF_2CF_3$$

**10.** Verbindungen nach Anspruch 1, nämlich

14α,17α-Ethano-11β-{4-[5-(pyrrolidin-1-yl)pentyloxy]phenyl}-1,3,5(10)-estratrien-3,17β-diol
14α,17α-Ethano-11β-{4-[5-(pentylthio)pentyloxy]phenyl}-1,3,5(10)-estratrien-3,17β-diol
14α,17α-Ethano-11β-{4-[5-(pentansulfinyl)pentyloxy]phenyl}-1,3,5(10)-estratrien-3,17β-diol
14α,17α-Ethano-11β-{4-[5-(pentansulfonyl)pentyloxy]phenyl }-1,3,5(10)-estratrien-3,17β-diol
14α,17α-Ethano-11β-{4-[5-(4,4,5,5,5-pentafluorpentylsulfinyl)pentyloxy]phenyl}-1,3,5(10)-estratrien-3,17β-diol
14α,17α-Ethano-11β-{4-[5-(2-pyridinmethylsulfonyl)pentyloxy]phenyl}-1,3,5(10)-estratrien-3,17β-diol
14α,17α-Ethano-11β-{4-[6-(4,4,5,5,5-pentafluorpentylthio)hexyloxy]-phenyl}-1,3,5(10)-estratrien-3,17β-diol
14α,17α-Ethano-11β-{4-[6-(4,4,5,5,5-pentafluorpentyl)sulfinyl]hexyloxy}phenyl-1,3,5(10)-estratrien-3,17β-diol
14α,17α-Ethano-11β-{4-[6-(4,4,5,5,5-pentafluorpentyl)sulfonyl]hexyloxy}phenyl-1,3,5(10)-estratrien-3,17β-diol
14α,17α-Ethano-11β-{4-[5-(2-pyridinmethylsulfinyl)pentyloxy]phenyl}-1,3,5(10)-estratrien-3,17β-diol
14α,17α-Ethano-11β-{4-[5-(4,4,5,5,5-pentafluorpentyl)sulfonyl]pentyloxy}phenyl-1,3,5(10)-estratrien-3,17β-diol
2-(6-(4-[10-{14,17-Ethano-3,17-dihydroxyestra-1,3,5(10)-trien-11β-yl}]-phenoxy)-hexy loxy)essigsäure-(N-butyl-N-methyl)-amid

**11.** Pharmazeutische Präparate, enthaltend mindestens eine Verbindung der allgemeinen Formel I gemäß Anspruch 1 sowie einen pharmazeutisch verträglichen Träger.

**12.** Verwendung der Verbindungen der allgemeinen Formel I zur Herstellung von Arzneimitteln.

**13.** Verfahren zur Herstellung der Verbindungen der allgemeinen Formel I, **dadurch gekennzeichnet, daß** eine Verbindung der allgemeinen Formel II

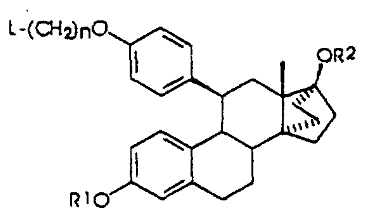

(II),

worin

$R^1$, $R^2$ und n die bereits in der allgemeinen Formel I angegebenen Bedeutungen haben und
L für eine Abgangsgruppe

steht,
wenn $R^3$ in der Verbindung der allgemeinen Formel I für eine Gruppierung $-(CH_2)_nNR^4R^5$ stehen soll,
mit einer Verbindung der allgemeinen Formel III

$$H\text{-}NR^4R^5 \qquad (III)$$

worin $R^4$ und $R^5$ die in der allgemeinen Formel I angegebene Bedeutung haben, oder wenn $R^3$ in der Verbindung der allgemeinen Formel I für eine Gruppierung $-(CH_2)_nS(O)_mR^6$- stehen soll,
mit einer das Thiolat-Anion der allgemeinen Formel IV

$$\text{-}SR6 \qquad (IV)$$

liefernden Verbindung, worin $R^6$ die in der allgemeinen Formel I angegebene Bedeutung hat, umgesetzt, und die erhaltene Thioverbindung anschließend gegebenenfalls entweder zur Sulfinyl- (m = 1) oder Sulfonylverbindung (m = 2) oxidiert, oder 14,17-Ethano-11β-(4-hydroxyphenyl)-17-hydroxyestra-4,9-dien-3-on (V)

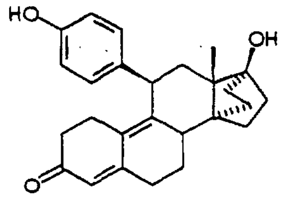

(V),

wenn $R^3$ in der Verbindung der allgemeinen Formel I für eine Gruppierung $-(CH_2)_nOCH_2CONR^4R^5$ stehen soll, mit einer Verbindung der allgemeinen Formel VI

$$L-(CH_2)_nOCH_2CONR^4R^5 \qquad (VI)$$

worin n, $R^4$ und $R^5$ die in der allgemeinen Formel I angegebene Bedeutung haben und L für eine Abgangsgruppe steht, zu einer Verbindung der allgemeinen Formel VII

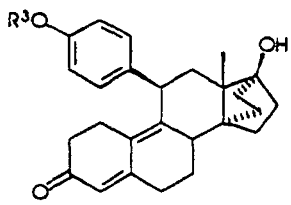

(VII),

umgesetzt und diese dann durch Aromatisierung des A-Ringes des Steroidgerüsts in eine Verbindung der allgemeinen Formel I, in der $R^1$ für ein Wasserstoffatom und $R^3$ für eine Gruppierung $-(CH_2)_nOCH_2CONR^4R^5$ steht, überführt wird.

**14.** Zwischenverbindungen der allgemeinen Formel II

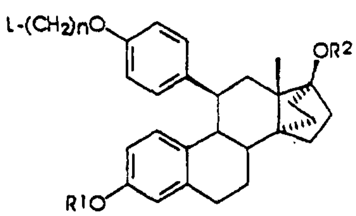

(II),

worin

$R^1$, $R^2$ und n die bereits in der allgemeinen Formel I angegebenen Bedeutungen haben und
L für eine Abgangsgruppe

steht.

**15.** Zwischenprodukte der allgemeinen Formel II nach Anspruch 14, worin $R^1$ und $R^2$ je für ein Wasserstoffatom, n für 5 oder 6 sowie L für eine Hydroxygruppe, ein Chlor-, Brom- oder Iodatom oder für eine Tosylgruppe stehen.

**16.** Zwischenprodukte der allgemeinen Formel II nach Anspruch 14, nämlich

14,17-Ethano-11β-(4-[5-chlorpentyloxy]phenyl)-estra-1,3,5(10)-trien-3,17-diol
14,17-Ethano-11β-(4-[5-iodpentyloxy]phenyl)-estra-1,3,5(10)-trien-3,17-diol
14,17-Ethano-11β-(4-[6-hydroxyhexyloxy]phenyl)-estra-1,3,5(10)-trien-3,17-diol
3-Benzyloxy-14,17-ethano-11β-(4-[6-hydroxyhexyloxy]phenyl)-estra-1,3,5(10)-trien-17-ol
3-Benzyloxy-14,17-ethano-11β-(4-[6-tosyloxyhexyloxy]phenyl)-estra-1,3,5(10)-trien-17-ol.

## Claims

**1.** 11β-Aryl-substituted 14,17-ethanoestratrienes of the general formula I

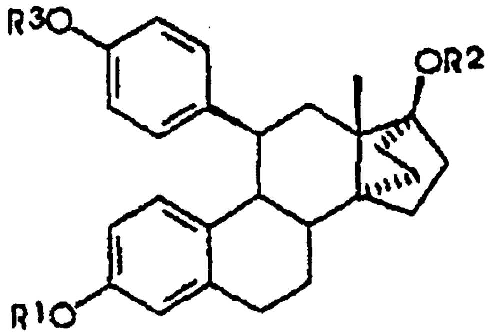

(I),

in which

$R^1$ stands for a hydrogen atom, a $C_1$-$C_{12}$-alkanoyl group, a benzoyl group, a straight-chain or branched-chain $C_1$-$C_{12}$-alkyl group, a $C_3$-$C_7$-cycloalkyl group or a $C_4$-$C_8$-alkylcycloalkyl group, which all can optionally be substituted,
$R^2$ stands for a hydrogen atom or an optionally substituted $C_1$-$C_{12}$-alkanoyl group and
$R^3$ stands for a group
$-(CH_2)_nNR^4R^5$ or $-(CH_2)_nOCH_2CONR^4R^5$ or $-(CH_2)_nS(O)_mR^6$,

in which

n is 4, 5, 6 or 7,
m is 0, 1 or 2,
$R^4$ or $R^5$, independently of one another, denote a hydrogen atom or a straight-chain or branched, optionally substituted $C_1$-$C_8$-alkyl group, which can also be partially or completely fluorinated, or
$R^4$ and $R^5$ together denote a methylene to pentamethylene group
$-(CH_2)_p-$ (p = 1-5), and
$R^6$ has the meaning of $R^4$ or $R^5$ or is an optionally substituted aryl or arylalkyl radical, where the alkyl radical therein can have up to 6 carbon atoms and the aryl radical, alone or in the arylalkyl radical, is a 5- or 6-membered monocyclic ring or a condensed, 8- to 10-membered ring system, and the aryl radical can have one or more heteroatoms selected from oxygen, nitrogen and sulphur.

**2.** 11β-Aryl-substituted 14,17-ethanoestratrienes according to Claim 1, in which $R^1$ is a hydrogen atom.

**3.** 11β-Aryl-substituted 14,17-ethanoestratrienes according to Claim 1, in which $R^2$ is a hydrogen atom.

**4.** 11β-Aryl-substituted 14,17-ethanoestratrienes according to Claim 1, in which $R^3$ stands for a group $-(CH_2)_nNR^4R^5$.

**5.** 11β-Aryl-substituted 14,17-ethanoestratrienes according to Claim 1, in which $R^3$ stands for a group $-(CH_2)_nOCH_2CONR^4R^5$.

**6.** 11β-Aryl-substituted 14,17-ethanoestratrienes according to Claim 1, in which $R^3$ stands for a group $-(CH_2)_nS(O)_mR^6$.

**7.** 11β-Aryl-substituted 14,17-ethanoestratrienes according to Claim 6, in which $R^6$ stands for an n-pentyl group or $-CH_2$-(2-pyridine) group, $-CH_2$-(3-pyridine) group or $-CH_2$-(4-pyridine) group.

**8.** 11β-Aryl-substituted 14,17-ethanoestratrienes according to Claim 6, in which $R^6$ stands for a 4,4,5,5,5-pentafluoropentyl group.

**9.** 11β-Aryl-substituted 14,17-ethanoestratrienes according to Claim 1, in which $R^3$ stands for one of the following substituents:

$$-(CH_2)_5S(CH_2)_4CH_3$$

$$-(CH_2)_5SO(CH_2)_4CH_3$$

$$-(CH_2)_5SO_2(CH_2)_4CH_3$$

$$-(CH_2)_5SO(CH_2)_3CF_2CF_3$$

$-(CH_2)_5SO_2-CH_2$-(2-pyridyl)

$-(CH_2)_5SO_2-CH_2$-(3-pyridyl)

$-(CH_2)_5SO_2-CH_2$-(4-pyridyl)

$$-(CH_2)_6-O-CH_2C(O)N(CH_3)C_4H_9$$

$$-(CH_2)_5SO_2(CH_2)_3CF_2CF_3$$

$-(CH_2)_5SO-CH_2$-(2-pyridyl)

$-(CH_2)_5SO-CH_2$-(3-Pyridyl)

$-(CH_2)_5SO-CH_2$-(4-pyridyl)

$-(CH_2)_5$-pyrrolidinyl

$$-(CH_2)_6S(CH_2)_3CF_2CF_3$$

$$-(CH_2)_6SO(CH_2)_3CF_2CF_3$$

$$-(CH_2)_6SO_2(CH_2)_3CF_2CF_3$$

**10.** Compounds according to Claim 1, namely

14α,17α-Ethano-11β-{4-[5-(pyrrolidin-1-yl)pentyloxy]-phenyl)-1,3,5(10)-oestratriene-3,17β-diol
14α,17α-ethano-11β-{4-[5-(pentylthio)pentyloxy]phenyl)-1,3,5(10)-oestratriene-3,17β-diol
14α,17α-ethano-11β-{4-[5-(pentanesulphinyl)pentyloxy]-phenyl}-1,3,5(10)-estratriene-3,17β-diol
14α,17α-ethano-11β-{4-[5-(pentanesulphonyl)pentyloxy]-phenyl)-1,3,5(10)-oestratriene-3,17β-diol
14α,17α-ethano-11β-{4-[5-(4,4,5,5,5-pentafluoropentylsulphinyl)pentyloxy]phenyl}-1,3,5(10)-oestratriene-3,17β-diol
14α,17α-ethano-11β-{4-[5-(2-pyridinomethylsulphonyl)-pentyloxy]phenyl}-1,3,5(10)-oestratriene-3,17β-diol
14α,17α-ethano-11β-{4-[6-(4,4,5,5,5-pentafluoropentyl-thio)hexyloxy]phenyl}-1,3,5(10)-oestratriene-3,17β-diol
14α,17α-ethano-11β-{4-[6-(4,4,5,5,5-pentafluoropentyl)-sulphinyl]hexyloxy}phenyl-1,3,5(10)-oestratriene-3,17β-diol
14α,17α-ethano-11β-{4-[6-(4,4,5,5,5-pentafluoropentyl)-sulphonyl]hexyloxy}phenyl-1,3,5(10)-oestratriene-3,17β-diol
14α,17α-ethano-11β-{4-[5-(2-pyridinomethylsulphinyl)-pentyloxy]phenyl}-1,3,5(10)-oestratriene-3,17β-diol
14α,17α-ethano-11β-{4-[5-(4,4,5,5,5-pentafluoropentyl)-sulphonyl]pentyloxy}phenyl-1,3,5(10)-oestratriene-3,17β-diol
2-(6-(4-[10-{14,17-ethano-3,17-dihydroxyoestra-1,3,5(10)-trien-11β-yl}]phenoxy)hexyloxy)acetic acid (N-butyl-N-methyl)-amide.

**11.** Pharmaceutical preparations that contain at least one compound of the general formula I according to Claim 1 and also a pharmaceutically compatible vehicle.

**12.** Use of the compounds of the general formula I for the production of pharmaceutical agents.

**13.** Process for the preparation of the compounds of the general formula I, **characterized in that** a compound of the general formula II

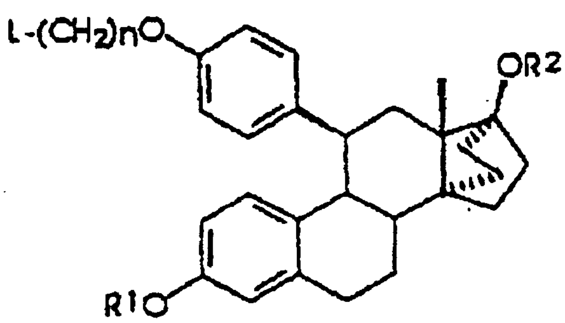

(II),

in which

$R^1$, $R^2$ and n have the meanings that are already indicated in the general formula I and L stands for a leaving group,
if $R^3$ in the compound of the general formula I is to stand for a group $-(CH_2)_nNR^4R^5$,

is reacted with a compound of the general formula III

$$H-NR^4R^5 \qquad (III)$$

in which $R^4$ and $R^5$ have the meaning that is indicated in the general formula I, or
if $R^3$ in the compound of the general formula I is to stand for a group $-(CH_2)_nS(O)_mR^6$,
is reacted with a compound that supplies the thiolate anion of the general formula IV

$$-SR6 \qquad (IV)$$

in which $R^6$ has the meaning that is indicated in the general formula I, and the thio compound that is obtained is then optionally either oxidized to the sulphinyl compound (m = 1) or sulphonyl compound (m = 2), or 14,17-ethanol-11β-(4-hydroxyphenyl)-17-hydroxyoestra-4,9-dien-3-one (V)

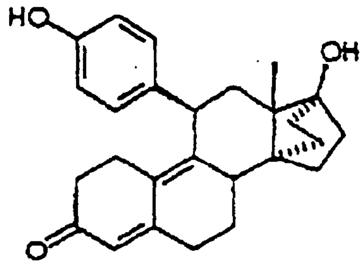

(V),

if $R^3$ in the compound of the general formula I is to stand for a group $-(CH_2)_nOCH_2CONR^4R^5$, is reacted with a compound of the general formula VI

$$L-(CH_2)_nOCH_2CONR^4R^5 \quad (VI)$$

in which n, $R^4$ and $R^5$ have the meaning that is indicated in the general formula I and L stands for a leaving group, to give a compound of the general formula VII

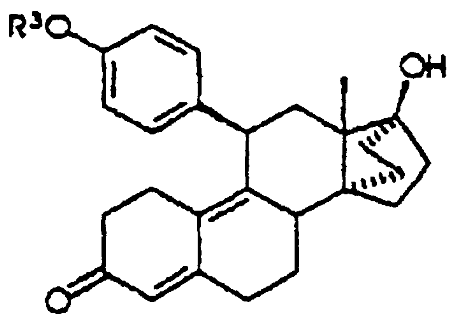

(VII),

and the latter is then converted by aromatization of the A ring of the steroid skeleton into a compound of the general formula I in which $R^1$ stands for a hydrogen atom and $R^3$ stands for a group $-(CH_2)_nOCH_2CONR^4R^5$.

**14.** Intermediate compounds of the general formula II

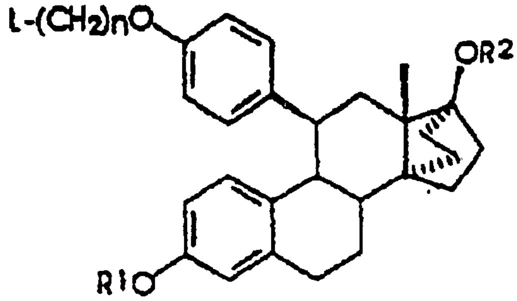

(II),

in which
$R^1$, $R^2$ and n have the meanings that are already indicated in the general formula I and L stands for a leaving group.

**15.** Intermediates of the general formula II according to Claim 14, in which $R^1$ and $R^2$ each stand for a hydrogen atom, n stands for 5 or 6, and L stands for a hydroxyl group, a chlorine, bromine or iodine atom or for a tosyl group.

**16.** Intermediates of the general formula II according to Claim 14, namely

14,17-ethano-11β-(4-[5-chloropentyloxy]phenyl)oestra-1,3,5(10)-trien-3,17-diol
14,17-ethano-11β-(4-[5-iodopentyloxy]phenyl)oestra-1,3,5(10)-trien-3,17-diol
14,17-ethano-11β-(4-[6-hydroxyhexyloxy]phenyl)oestra-1,3,5(10)-trien-3,17-diol
3-benzyloxy-14,17-ethano-11β-(4-[6-hydroxyhexyloxy]phenyl)oestra-1,3,5(10)-trien-3,17-ol
3-benzyloxy-14,17-ethano-11β-(4-[6-tosyloxyhexyloxy]-phenyl)oestra-1,3,5(10)-trien-3,17-ol.

## Revendications

**1.** 14,17-éthanoestratriènes substitués par un groupe aryle en 11β, de formule générale I

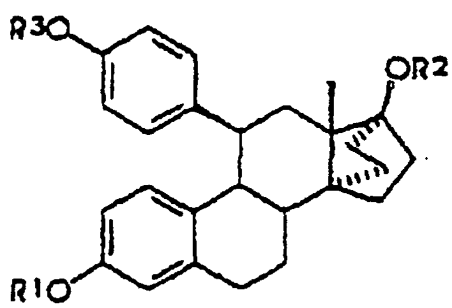

(I).

dans laquelle

- $R^1$ représente un atome d'hydrogène, un groupe alcanoyle en $C_1$-$C_{12}$, un groupe benzoyle, un groupe alkyle en $C_1$-$C_{12}$ à chaîne droite ou ramifiée, un groupe cycloalkyle en $C_3$-$C_7$ ou un groupe alkylcycloalkyle en $C_4$-$C_8$, qui peuvent tous être éventuellement substitués,
- $R^2$ représente un atome d'hydrogène ou un groupe alcanoyle en en $C_1$-$C_{12}$ éventuellement substitué et
- $R^3$ représente un groupement $-(CH_2)_nNR^4R^5$ ou $-(CH_2)_nOCH_2CONR^4R^5$ ou $-(CH_2)_nS(O)_mR^6$
  où
  - n est 4, 5, 6 ou 7,
  - m est 0, 1 ou 2,
  - $R^4$ ou $R^5$ représente, indépendamment l'un de l'autre, un atome d'hydrogène ou un groupe alkyle en $C_1$-$C_8$ à chaîne droite ou ramifiée, éventuellement substitué, qui peut également être partiellement ou totalement fluoré, ou
  - $R^4$ et $R^5$ représentent ensemble un groupe méthylène à pentaméthylène $-(CH_2)_p-$ (p = 1-5), et
  - $R^6$ a la signification de $R^4$ ou $R^5$ ou est un radical aryle ou arylalkyle éventuellement substitué, le radical alkyle dans ce dernier pouvant avoir jusqu'à 6 atomes de carbone et le radical aryle, seul ou dans le radical arylalkyle, étant un cycle monocyclique à 5 ou 6 chaînons ou un système cyclique condensé à 8-10 chaînons, et le radical aryle pouvant comporter un ou plusieurs hétéroatomes choisis parmi les atomes d'oxygène, d'azote et de soufre.

**2.** 14,17-éthanoestratriènes substitués par un groupe aryle en 11β, selon la revendication 1, dans lesquels $R^1$ est un atome d'hydrogène.

**3.** 14,17-éthanoestratriènes substitués par un groupe aryle en 11β, selon la revendication 1, dans lesquels $R^2$ est un atome d'hydrogène.

**4.** 14,17-éthanoestratriènes substitués par un groupe aryle en 11β, selon la revendication 1, dans lesquels $R^3$ représente un groupement $-(CH_2)_nNR^4R^5$.

**5.** 14,17-éthanoestratriènes substitués par un groupe aryle en 11β, selon la revendication 1, dans lesquels $R^3$ représente un groupement $-(CH_2)_nOCH_2CONR^4R^5$.

**6.** 14,17-éthanoestratriènes substitués par un groupe aryle en 11β, selon la revendication 1, dans lesquels $R^3$ représente un groupement $-(CH_2)_nS(O)_mR^6$.

**7.** 14,17-éthanoestratriènes substitués par un groupe aryle en 11β, selon la revendication 6, dans lesquels $R^6$ représente un groupe n-pentyle ou $-CH_2$-(2-pyridine), $-CH_2$-(3-pyridine) ou $-CH_2$-(4-pyridine).

**8.** 14,17-éthanoestratriènes substitués par un groupe aryle en 11β, selon la revendication 6, dans lesquels $R^6$ représente un groupe 4,4,5,5,5-pentafluoropentyle.

**9.** 14,17-éthanoestratriènes substitués par un groupe aryle en 11β, selon la revendication 1, dans lesquels $R^3$ représente l'un des substituants suivants :

$$-(CH_2)_5S(CH_2)_4CH_3$$

$$-(CH_2)_5SO(CH_2)_4CH_3$$

$$-(CH_2)_5SO_2(CH_2)_4CH_3$$

$$-(CH_2)_5SO(CH_2)_3CF_2CF_3$$

$-(CH_2)_5SO_2-CH_2$-(2-pyridyle)

$-(CH_2)_5SO_2-CH_2$-(3-pyridyle)

$-(CH_2)_5SO_2-CH_2$-(4-pyridyle)

$$-(CH_2)_6-O-CH_2C(O)N(CH_3)C_4H_9$$

$$-(CH_2)_5SO_2-(CH_2)_3CF_2CF_3$$

$-(CH_2)_5SO-CH_2$-(2-pyridyle)

$-(CH_2)_5SO-CH_2$-(3-pyridyle)

$-(CH_2)_5SO-CH_2$-(4-pyridyle)

$-(CH_2)_5$-pyrrolidinyle

$-(CH_2)_6S(CH_2)_3CF_2CF_3$

$-(CH_2)_6SO(CH_2)_3CF_2CF_3$

$-(CH_2)_6SO_2(CH_2)_3CF_2CF_3$.

**10.** Composés selon la revendication 1, à savoir

14α,17α-éthano-11β-{4-[5-(pyrrolidin-1-yl)pentyloxy]phényl}-1,3,5(10)-estratriène-3,17β-diol
14α,17α-éthano-11β-{4-[5-(pentylthio)pentyloxy]phényl}-1,3,5(10)estratriène-3,17β-diol
14α,17α-éthano-11β-{4-[5-(pentanesulfinyl)pentyloxy]phényl}-1,3,5(10)-estratriène-3,17β-diol
14α,17α-éthano-11β-{4-[5-(pentanesulfonyl)pentyloxy]phényl}-1,3,5(10)-estratriène-3,17β-diol
14α,17α-éthano-11β-{4-[5-(4,4,5,5,5-pentafluoropentylsulfinyl)pentyloxy]phényl}-1,3,5(10)estratriène-3,17β-diol
14α,17α-éthano-11β-{4-[5-(2-pyridineméthylsulfonyl)pentyloxy]phényl}1,3,5(10)estratriène-3,17β-diol
14α,17α-éthano-11β-{4-[6-(4,4,5,5,5-pentafluoropentylthio)hexyloxy]phényl}1,3,5(10)estratriène-3,17β-diol
14α,17α-éthano-11β-{4-[6-(4,4,5,5,5-pentafluoropentyl)sulfinyl)hexyloxy}phényl-1,3,5(10)estratriène-3,17β-diol
14α,17α-éthano-11β-{4-[6-(4,4,5,5,5-pentafluoropentyl)sulfonyl]hexyloxy}phényl-1,3,5(10)estratriène-3,17β-diol
14α,17α-éthano-11β-{4-[5-(2-pyridinométhylsulfinyl)pentyloxy]phényl}-1,3,5(10)estratriène-3,17β-diol
14α,17α-éthano-11β-{4-[5-(4,4,5,5,5-pentafluoropentyl)sulfonyl]pentyloxy}phényl-1,3,5(10)estratriène-3,17β-diol
2-(6-(4-[10-{14,17-éthano-3,17-dihydroxyestra-1,3,5(10)-triène-11β-yl}]phénoxy)hexyloxy)-(N-butyl-N-méthyl)acétamide.

**11.** Préparations pharmaceutiques contenant au moins un composé de formule générale I selon la revendication 1, ainsi qu'un véhicule pharmaceutiquement acceptable.

**12.** Utilisation des composés de formule générale I, pour la fabrication de médicaments.

**13.** Procédé pour la préparation des composés de formule générale I, **caractérisé en ce qu'**on fait réagir un composé de formule générale II

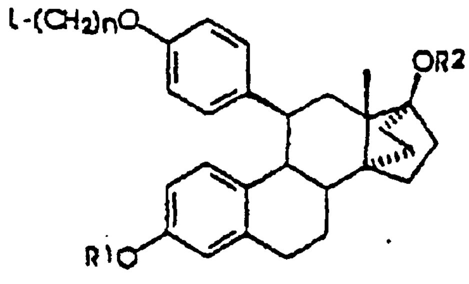

(II).

dans laquelle

$R^1$, $R^2$ et n ont les significations déjà indiquées dans la formule générale I, et
L représente un groupe partant,
si $R^3$ dans le composé de formule générale I doit représenter un groupement $-(CH_2)_nNR^4R^5$,

avec un composé de formule générale III

$$H\text{-}NR^4R^5 \quad (III)$$

dans laquelle $R^4$ et $R^5$ ont les significations données dans la formule générale I, ou si $R^3$ dans le composé de formule générale I doit représenter un groupement $-(CH_2)_nS(O)_mR^6$, avec un composé fournissant un anion thiolate de formule générale IV

$$-SR^6 \quad (IV)$$

dans laquelle $R^6$ a la signification indiquée dans la formule générale I, et le composé thio obtenu est ensuite éventuellement oxydé soit en le composé sulfinyle (m = 1), soit en le composé sulfonyle (m = 2), ou on fait réagir la 14,17-éthano-11β-(4-hydroxyphényl)-17-hydroestra-4,9-dién-3-one (V)

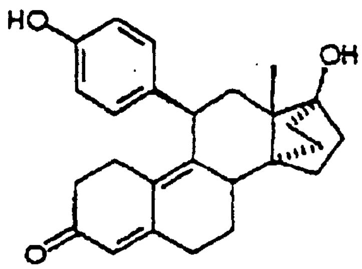

(V),

si $R^3$ dans le composé de formule générale I doit représenter un groupement $-(CH_2)_nOCH_2CONR^4R^5$, avec un composé de formule générale VI

$$L\text{-}(CH_2)_nOCH_2CONR^4R^5 \quad (VI)$$

dans laquelle n, $R^4$ et $R^5$ ont les significations données dans la formule générale I, et L représente un groupe partant, pour aboutir à un composé de formule générale VII

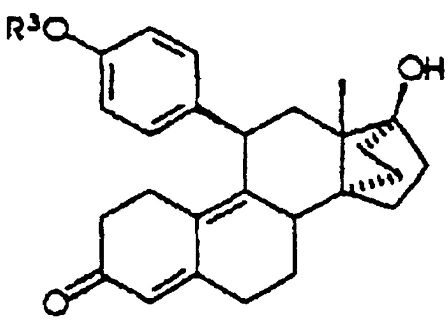

(VII),

et ce dernier est ensuite converti, par aromatisation du cycle A du squelette stéroïdien, en un composé de formule générale I dans lequel $R^1$ représente un atome d'hydrogène et $R^3$ représente un groupement $-(CH_2)_nOCH_2CONR^4R^5$.

**14.** Composés intermédiaires de formule générale II

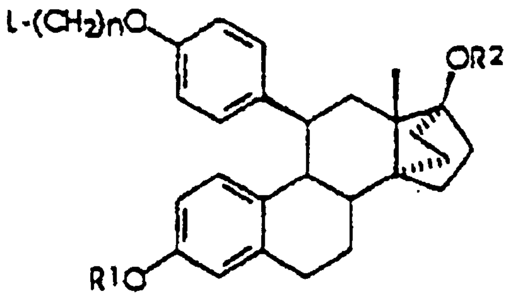

(II).

dans laquelle

$R^1$, $R^2$ et n ont les significations déjà indiquées dans la formule générale I et
L représente un groupe partant.

**15.** Produits intermédiaires de formule générale II selon la revendication 14, dans lesquels $R^1$ et $R^2$ représentent chacun un atome d'hydrogène, n représente 5 ou 6 et L représente un groupe hydroxy, un atome de chlore, de brome ou d'iode ou un groupe tosyle.

**16.** Produits intermédiaires de formule générale II selon la revendication 14, à savoir

14,17-éthano-11β-(4-[5-chloropentyloxy)phényl)estra-1,3,5(10)-triène-3,17-diol
14,17-éthano-11β-(4-[5-iodopentyloxy]phényl)estra-1,3,5(10)-triène-3,17-diol
14,17-éthano-11β-(4-[6-hydroxyhexyloxy]phényl)estra-1,3,5(10)-triène-3,17-diol
3-benzyloxy-14,17-éthano-11β-(4-[6-hydroxyhexyloxy]phényl)estra-1,3,5(10)-triène-17-ol
3-benzyloxy-14,17-éthano-11β-(4-[6-tosyloxyhexyloxy]phényl)estra-1,3,5(10)-triène-17-ol.

Figur 1

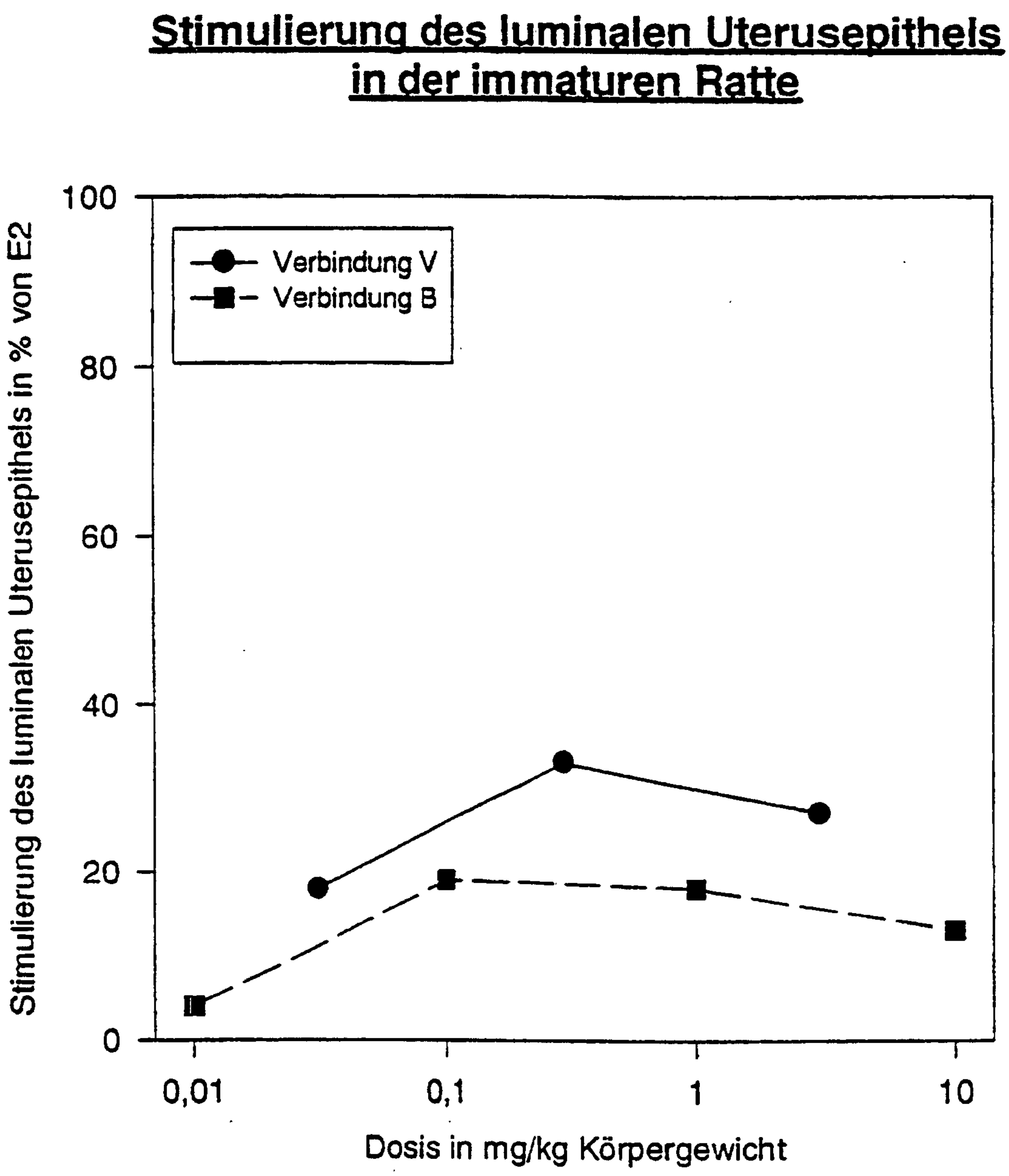
Stimulierung des luminalen Uterusepithels
in der immaturen Ratte
Stimulierung des luminalen Uterusepithels in % von E2
100
80
60
40
20
0
Verbindung V
Verbindung B
0,01
0,1
1
10
Dosis in mg/kg Körpergewicht

Figur 2

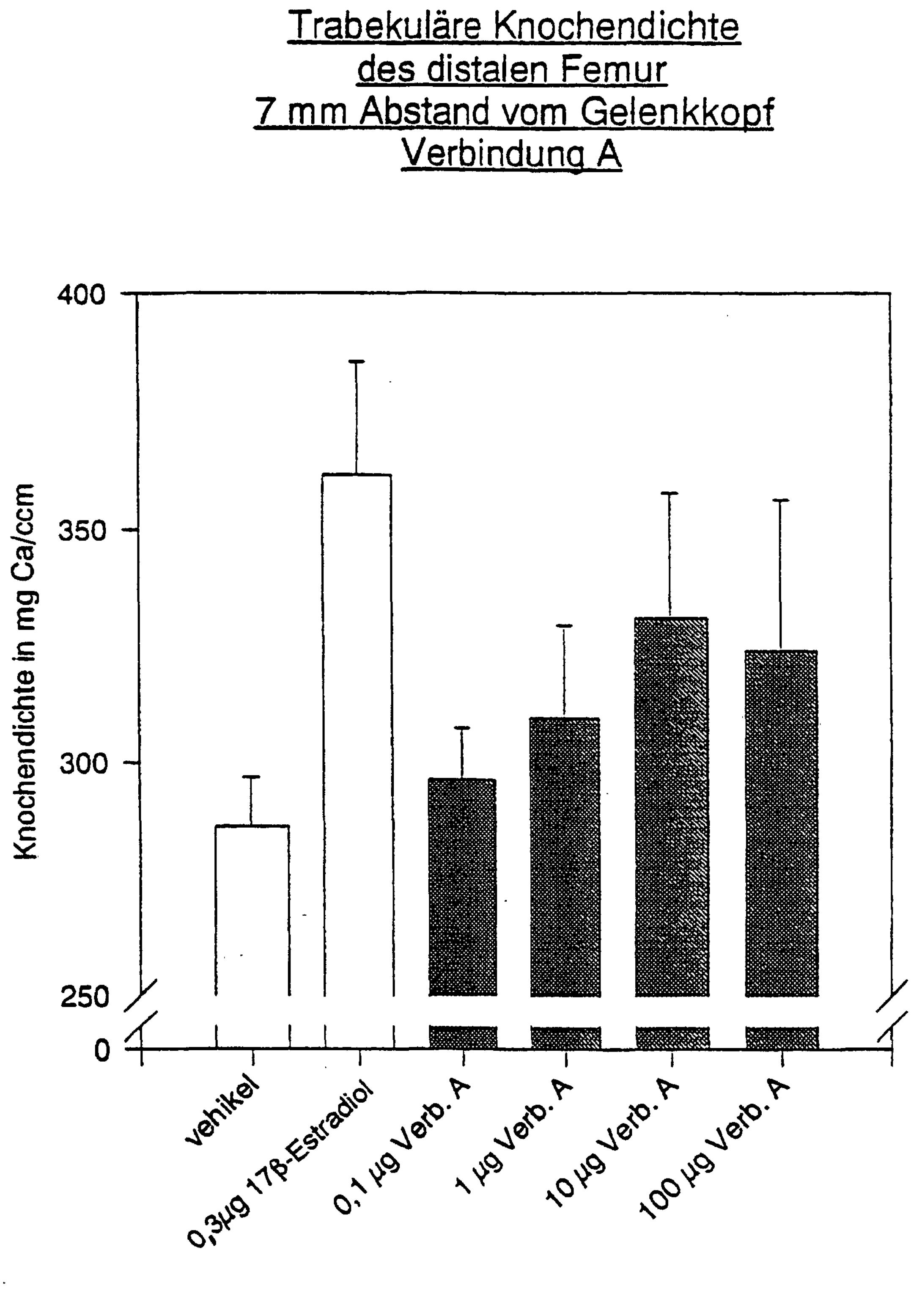
Trabekuläre Knochendichte
des distalen Femur
7 mm Abstand vom Gelenkkopf
Verbindung A
Knochendichte in mg Ca/ccm
400
350
300
250
0
vehikel
0,3µg 17β-Estradiol
0,1 µg Verb. A
1 µg Verb. A
10 µg Verb. A
100 µg Verb. A

Figur 3

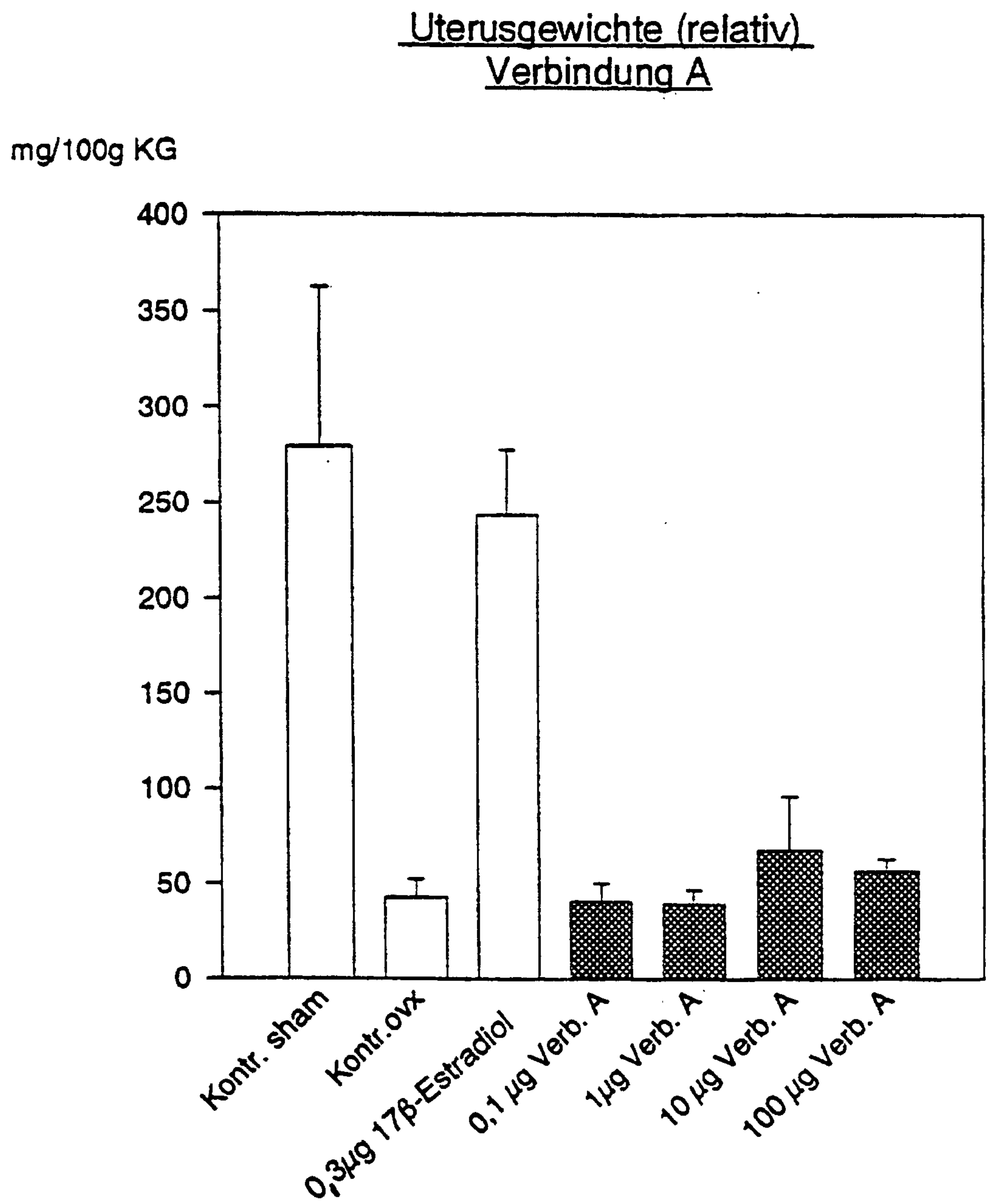
Uterusgewichte (relativ)
Verbindung A
mg/100g KG
400
350
300
250
200
150
100
50
0
Kontr. sham
Kontr.ovx
0,3µg 17β-Estradiol
0,1 µg Verb. A
1µg Verb. A
10 µg Verb. A
100 µg Verb. A

Figur 4

Trabekuläre Knochendichte
der proximalen Tibia
5 mm Abstand vom Gelenkkopf
Verbindung A

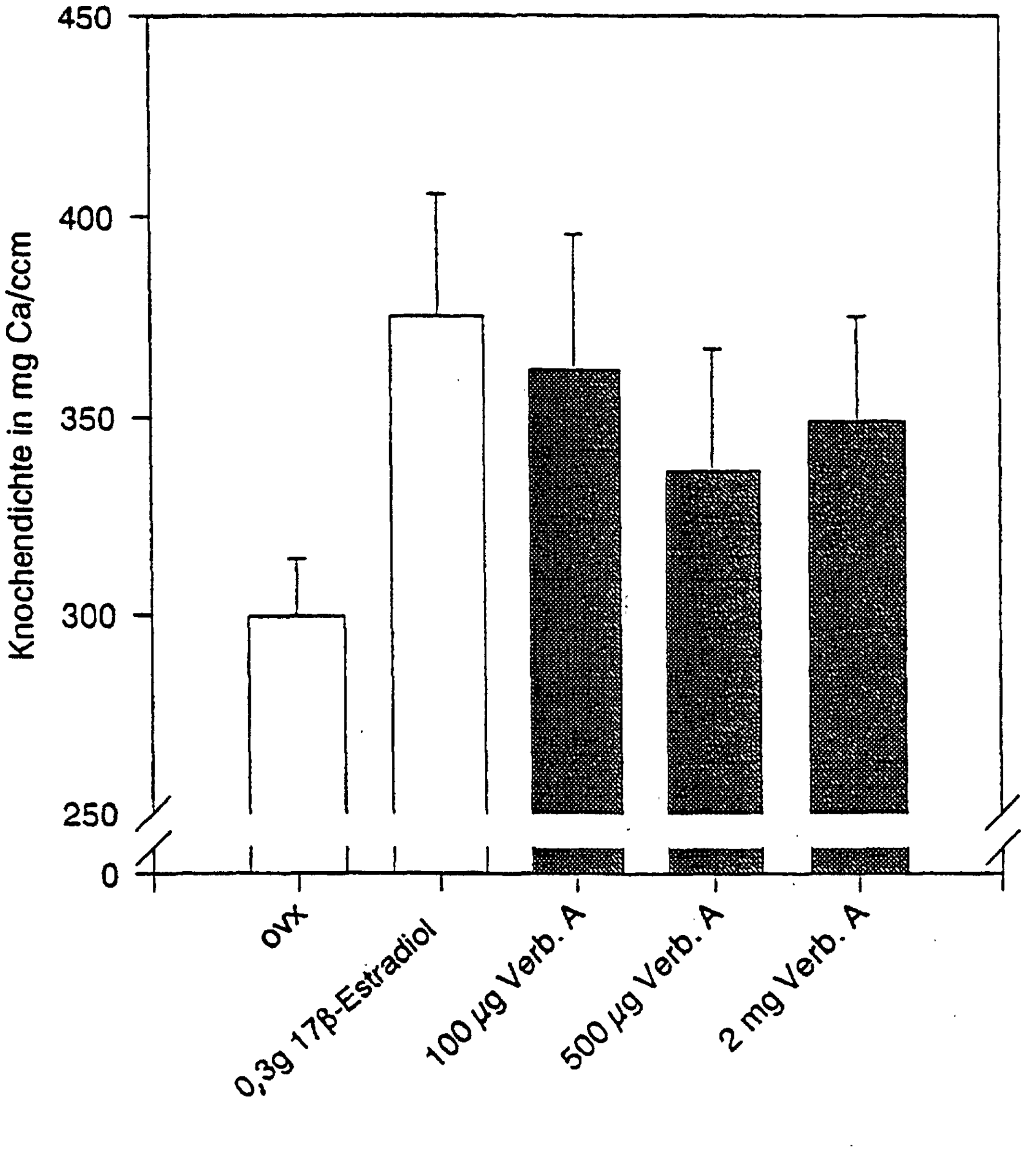

Figur 5

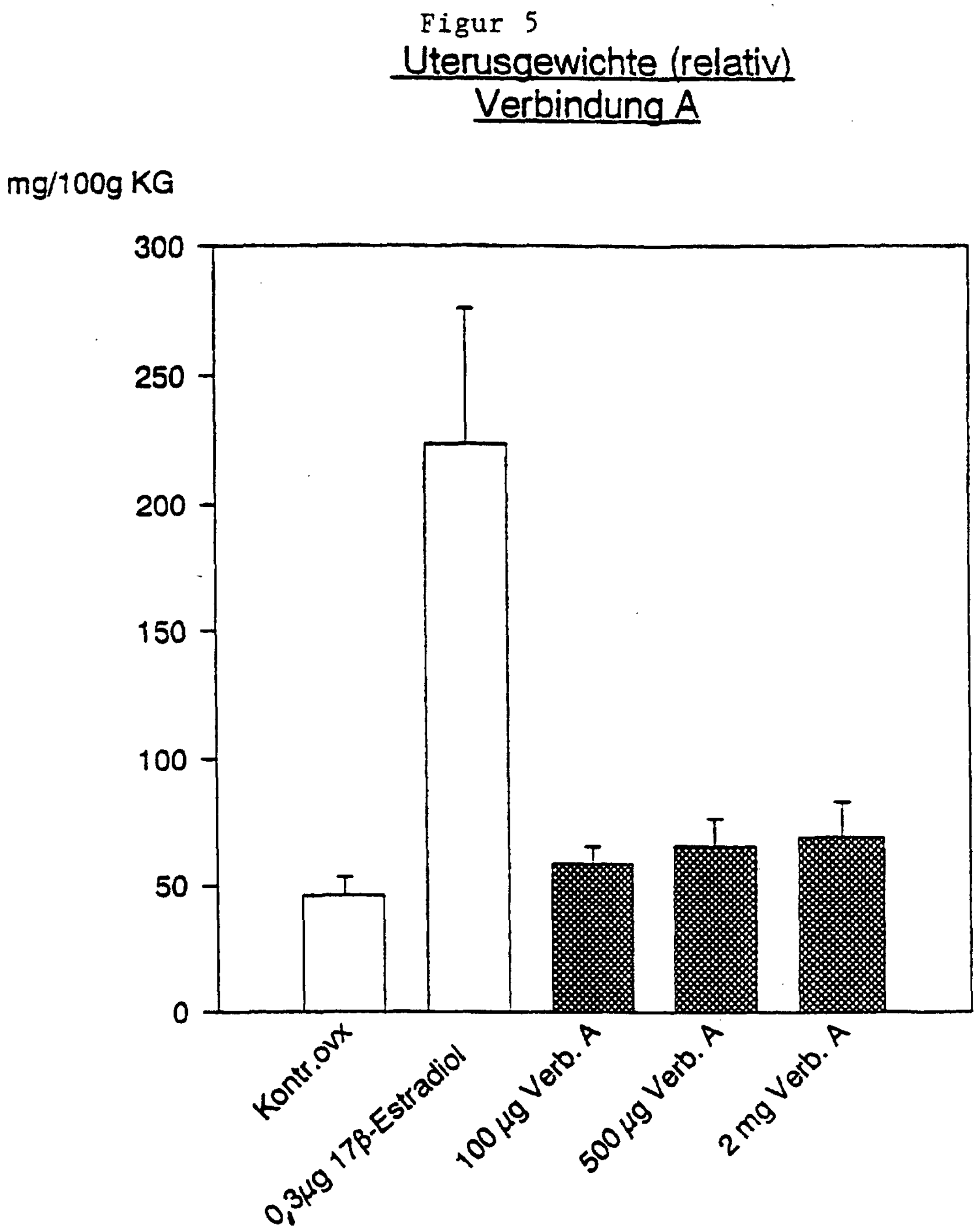
Uterusgewichte (relativ)
Verbindung A
mg/100g KG
300
250
200
150
100
50
0
Kontr.ovx
0,3µg 17β-Estradiol
100 µg Verb. A
500 µg Verb. A
2 mg Verb. A

Figur 6

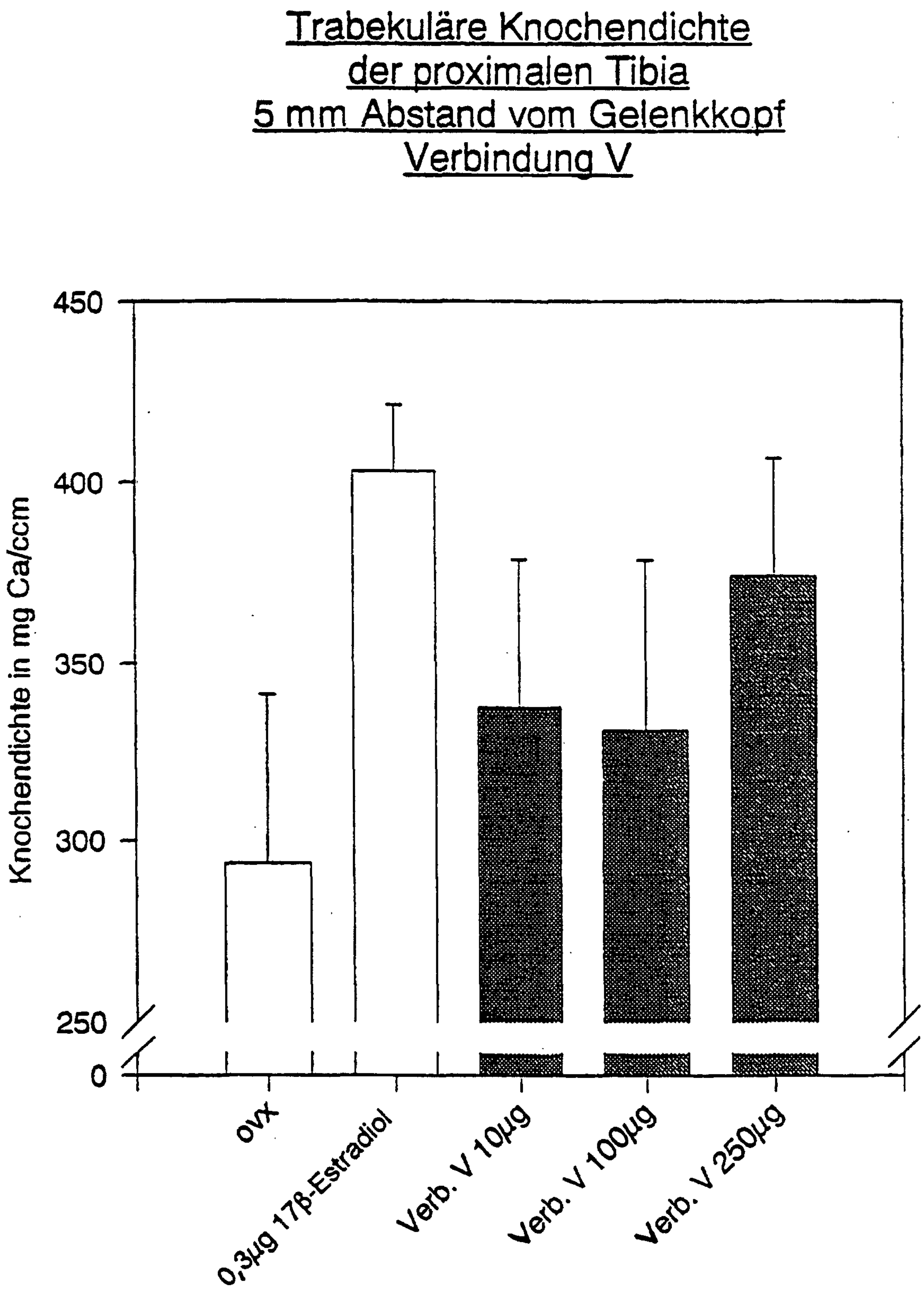
Trabekuläre Knochendichte
der proximalen Tibia
5 mm Abstand vom Gelenkkopf
Verbindung V
Knochendichte in mg Ca/ccm
450
400
350
300
250
0
ovx
0,3µg 17β-Estradiol
Verb. V 10µg
Verb. V 100µg
Verb. V 250µg

Figur 7

**Dissoziation Knochenwirkung/ Uteruswirkung**